# EUROPEAN PATENT APPLICATION

(11) **EP 4 696 254 A2**
(43) Date of publication of application: **18.02.2026**
(21) Application number: 25195661.1
(22) Date of filing: 13.08.2025
(51) Int. Cl.: A61B 17/16

(54) **SURGICAL ASSEMBLY FOR COUPLING TO A SURGICAL ACCESSORY TOOL**

(30) Priority: 13.08.2024 US 202463682538 P
(71) Applicant: Stryker Corporation, Portage, MI 49002 (US)
(72) Inventor: FLATT, James E., Kalamazoo, MI 49001-6139 (US); MALLERY, Erika Grace, Kalamazoo, MI 49009 (US); KRAMER, Ian, Kalamazoo, MI 49008 (US); BASNEY, Emily, Portage, MI 49024-5079 (US)
(74) Representative: Schott, Jakob Valentin

(57) **Abstract**

A surgical assembly for coupling to a surgical accessory tool. The surgical assembly includes a driving member. The driving member is rotatable about an axis. The driving member defines a bore for receiving the tool and an opening. The driving member has a planar surface defining the opening. The planar surface faces the axis and is disposed at a non-zero angle relative to the axis. The surgical assembly also includes a lock assembly to secure the tool to the driving member. The lock assembly includes a retention member moveably disposed in the opening. In a first position, a first volume of the retention member is disposed in the bore. In a second position, a second volume of the retention member less than the first volume is disposed in the bore. A biasing member urges the retention member toward the first position to engage the tool.

## Description

### CROSS-REFERENCE TO RELATED APPLICATIONS

The subject patent application claims priority to and all the benefits of U.S. Provisional Patent Application No. 63/682,538 filed on August 13, 2024, the disclosure of which is hereby incorporated by reference in their entirety.

### BACKGROUND

Conventional medical and surgical procedures routinely involve the use of surgical tools and instruments which allow surgeons to approach and manipulate surgical sites. By way of non-limiting example, rotary instruments such as handheld drills are commonly utilized in connection with orthopedic procedures to address various musculoskeletal conditions, such as trauma, sports injuries, degenerative diseases, joint reconstruction, and the like. In procedures where handheld drills or similar surgical instruments are employed, rotational torque selectively generated by an actuator (e.g., an electric motor) is used to rotate drive shafts of a releasably-attachable surgical attachment at different speeds.

While handheld surgical instruments are routinely utilized to assist in the performance of a variety of different types of medical and/or surgical procedures, there is a need in the art to continuously improve such handheld surgical instruments.

### SUMMARY

One general aspect of the present disclosure provides a surgical assembly for coupling to a surgical accessory tool. The surgical assembly includes a driving member extending along an axis from a proximal end to a distal end. The driving member is rotatable about an axis and configured to transfer torque to the surgical accessory tool. The driving member defines: (i) a receiving bore extending along the axis for receiving the surgical accessory tool, (ii) an opening extending transverse to and partially into the receiving bore, and (iii) a cavity in communication with the opening. The surgical assembly also includes a lock assembly for securing the surgical accessory tool to the driving member. The lock assembly may include a retention member partially disposed in the opening of the driving member for movement between a plurality of retention member positions including, a first position where a first volume of the retention member is disposed in the receiving bore of the driving member, and a second position where a second volume of the retention member is disposed in the receiving bore of the driving member. The second volume is less than the first volume. The assembly also includes a retention biasing member at least partially disposed in the cavity of the driving member and configured to urge the retention member towards the first position for engaging the surgical accessory tool.

Another aspect of the present disclosure provides a surgical assembly for coupling to a surgical accessory tool. The surgical assembly also includes a driving member extending along an axis from a proximal end to a distal end. The driving member is rotatable about an axis and configured to transfer torque to the surgical accessory tool. The driving member defines (i) a receiving bore extending along the axis for receiving the surgical accessory tool, and (ii) an opening extending transverse to and partially into the receiving bore. The driving member has a planar surface at least partially defining the opening. The planar surface faces the axis and is disposed at an angle between one and thirty degrees relative to the axis. The surgical assembly also includes a lock assembly for securing the surgical accessory tool to the driving member. The lock assembly may include a retention member partially disposed in the opening of the driving member for movement between a plurality of retention member positions including, a first position where a first volume of the retention member is disposed in the receiving bore of the driving member, and a second position where a second volume of the retention member is disposed in the receiving bore of the driving member. The second volume is less than the first volume. The surgical assembly also includes a retention biasing member coupled to the driving member and configured to urge the retention member towards the first position for engaging the surgical accessory tool.

Another aspect of the present disclosure provides a surgical system. The surgical system includes a surgical accessory tool including at least one flat region and a groove opposite the flat region. The system also includes a surgical assembly adapted for removably coupling with the surgical accessory tool. The surgical assembly may include a driving member extending along an axis from a proximal end to a distal end. The driving member is rotatable about an axis and configured to transfer torque to the surgical accessory tool. The driving member defines: (i) a receiving bore extending along the axis for receiving the surgical accessory tool, and (ii) an opening extending transverse to and partially into the receiving bore. The surgical assembly also includes a drive element coupled to the driving member and disposed at least partially within the bore of the driving member opposite the axis from the opening of the driving member to engage the flat region of the surgical accessory tool to transmit torque to the surgical accessory tool. The surgical assembly also includes a lock assembly coupled to the driving member to axially secure the surgical accessory tool to the driving member. The lock assembly may include a retention member partially disposed in the opening of the driving member for movement between a plurality of retention member positions including, a first position where a first volume of the retention member is disposed in the receiving bore of the driving member, and a second position axially proximal relative to the first position, where second volume of the retention member is disposed in the receiving bore of the driving member. The second volume is less than the first volume. A portion of the retention member is disposed in the groove of the surgical accessory tool to axially secure the surgical accessory tool to the driving member. The lock assembly also includes a retention biasing member coupled to the driving member and configured to urge the retention member towards the first position.

Another aspect of the present disclosure provides a surgical system. The surgical system includes a surgical accessory tool including at least one flat region. The surgical system also includes a surgical assembly adapted for removable coupling with the surgical accessory tool. The surgical assembly may include a driving member extending along an axis from a proximal end to a distal end. The driving member is rotatable about an axis and configured to transfer torque to the surgical accessory tool. The driving member defines: (i) a receiving bore extending along the axis for receiving the surgical accessory tool, and (ii) an opening extending transverse to and partially into the receiving bore. The surgical assembly also includes a lock assembly coupled to the driving member to axially secure the surgical accessory tool to the driving member. The lock assembly includes a retention member partially disposed in the opening of the driving member for movement between a plurality of retention member positions including, a first position where a first volume of the retention member is disposed in the receiving bore of the driving member, and a second position where a second volume of the retention member is disposed in the receiving bore of the driving member. The second volume is less than the first volume. A portion of the retention member abuts the flat region of the surgical accessory tool to axially secure the surgical accessory tool to the driving member. The surgical assembly also includes a retention biasing member coupled to the driving member and configured to urge the retention member towards the first position

Any of the above aspects can be combined in full or in part. Any features of the above aspects can be combined in full or in part.

### BRIEF DESCRIPTION OF THE DRAWINGS

Advantages of the present disclosure will be readily appreciated as the same becomes better understood by reference to the following detailed description when considered in connection with the accompanying drawings.
FIG. 1A is a front perspective view of a sagittal saw attachment and a rotary surgical handpiece including the same.
FIG. 1B is a front perspective view of the sagittal saw attachment spaced from the rotary surgical handpiece.
FIG. 2 is a front perspective view of the sagittal saw attachment including a blade mount and a drive assembly.
FIG. 3 is a cross-sectional representation of sagittal saw attachment revealing the blade mount and the drive assembly.
FIG. 4 is a side view of the drive assembly and the blade mount.
FIG. 5 is a bottom perspective view of the drive assembly and the blade mount.
FIG. 6 is an exploded view of the drive assembly and the blade mount.
FIG. 7 is a perspective view of a sagittal saw system including a saw blade and a blade mount for releasably receiving the saw blade.
FIG. 8A is an exploded view of one example of the sagittal saw system of FIG. 7 including a cylindrical retention member.
FIG. 8B is an exploded view of one example of the sagittal saw system of FIG. 7 including a spherical retention member.
FIG. 9 is a partial top view of the sagittal saw system of FIG. 7.
FIGS. 10A-10E are cross-sectional representations of the sagittal saw system illustrating a sequence of inserting the saw blade into the blade mount.
FIGS. 11A-11C are cross-sectional representations of the sagittal saw system illustrating a sequence of actuating a release member and removing the saw blade from the blade mount.
FIG. 12 is a front perspective view of a surgical attachment and a rotary surgical handpiece including the same.
Figure 13 is a front perspective view of the surgical attachment.
Figure 14 is a cross-section view of the surgical attachment.
Figure 15 is a cross-section view of the surgical attachment in a disengaged position.
Figure 16 is a cross-section view of the surgical attachment in an engaged position.
Figure 17 is a cross-section view of the surgical attachment with a first and a second biasing member.
Figure 18 is an explode front perspective view of the surgical attachment with the first and second biasing members.
Figure 19 is an exploded rear perspective view of the surgical attachment with the first and second biasing members.
Figure 20 is a cross-section view of the surgical attachment with the first biasing member.
Figure 21 is a front, cross-sectional view showing a flat region of the surgical accessory tool engaged with a retention member.
Figure 22 is a perspective view of a second implementation of a surgical attachment coupled to a handheld surgical driver.
Figure 23 is a perspective view of the second implementation of the surgical attachment.
Figure 24 is an exploded view of the second implementation of the surgical attachment.
Figure 25 is a front elevation view of the second implementation of the surgical attachment.
Figure 26 is a section view of the second implementation of the surgical attachment taken along lines 26-26 of Figure 25.
Figure 27 is a section view of the second implementation of the surgical attachment taken along lines 27-27 of Figure 25.
Figure 28 is a section view of the second implementation of the surgical attachment with a surgical accessory tool coupled thereto.
Figure 29 is a perspective view of a third implementation of a surgical attachment.
Figure 30 is a partial perspective view of the third implementation of the surgical attachment.
Figure 31 is a section view of the third implementation of the surgical attachment.
Figure 32 is a perspective view of a fourth implementation of the surgical attachment with a surgical accessory tool coupled thereto.
Figure 33 is an exploded view of the fourth implementation of the surgical attachment with the surgical accessory tool coupled thereto.
Figure 34 is a section view of the fourth implementation of the surgical attachment.
Figure 35 is a section view of the fourth implementation of the surgical attachment with the surgical accessory coupled thereto.

### DETAILED DESCRIPTION

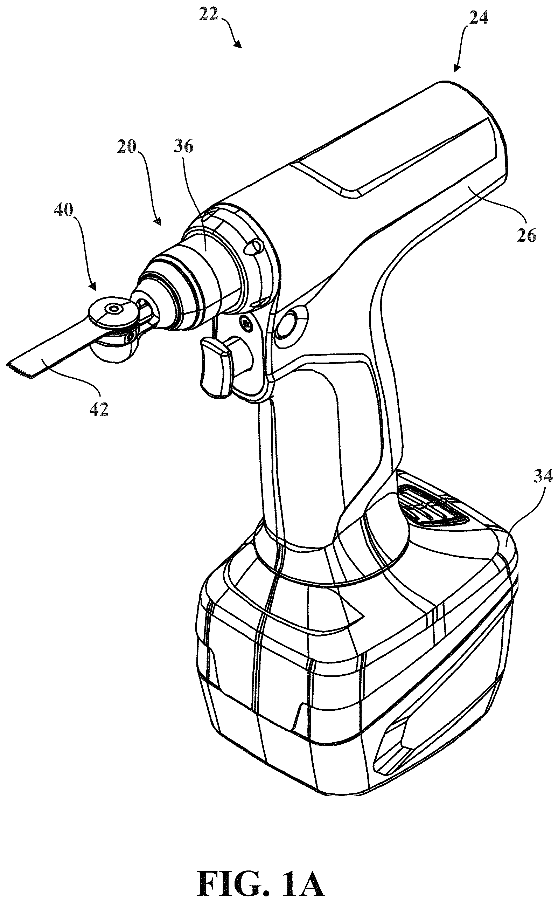

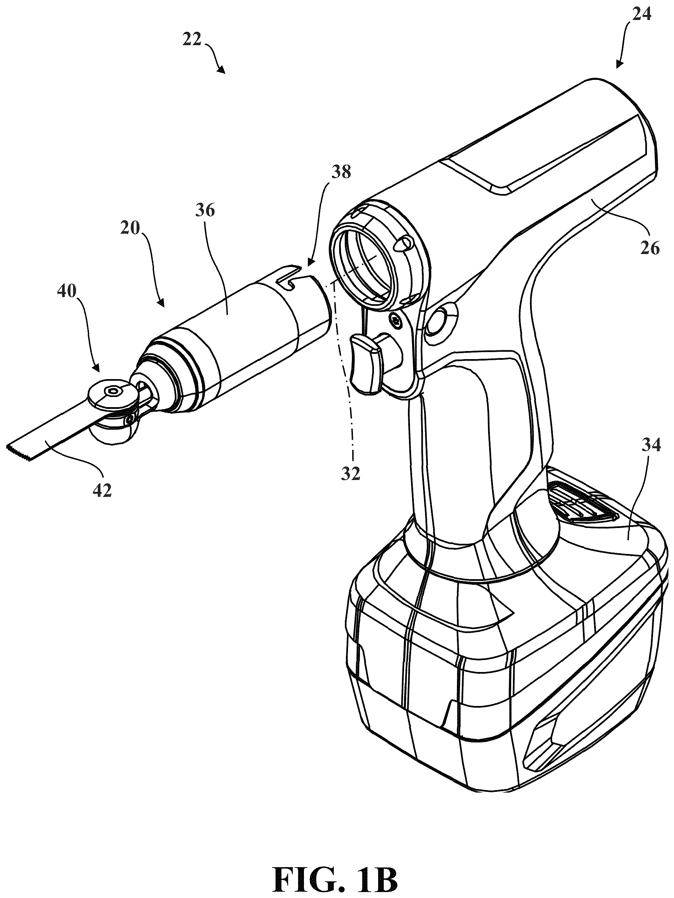

Referring now to the drawings, wherein like numerals indicate like parts throughout the several views, FIGS. 1A and 1B illustrate a sagittal saw attachment 20 according to the present disclosure and a surgical sagittal saw 22 including the same.

The surgical sagittal saw 22 includes a rotary surgical handpiece 24. The rotary surgical handpiece 24 includes a body 26 and a motor 28 disposed within the body 26. The motor 28 includes an output shaft 30 configured to be rotated about an output axis 32 in response to actuation of the motor 28. Beyond the structure described above, the configuration of the rotary surgical handpiece 24 is not particularly limited for the purposes of this disclosure. For example, the rotary surgical handpiece 24 may be an inline rotary surgical handpiece 24 or a rotary surgical handpiece 24 having a pistol grip. Exemplary rotary surgical handpieces 24 can be found in U.S. Pat. Pub. No. 2007/0021766 and U.S. Patent No. 10,537,339, which are incorporated by reference in their entirety herein. In some implementations, the rotary surgical handpiece 24 may further comprise additional components. For example, the rotary surgical handpiece 24 may be cordless (i.e., include a battery 34 for powering the rotary surgical handpiece 24) or may include a console (not shown) to power the rotary surgical handpiece 24 and control the rotary surgical handpiece 24 (e.g., via a footswitch). Additionally, the rotary surgical handpiece 24 further comprise an irrigation system (not shown), a navigation system (not shown), and the like.

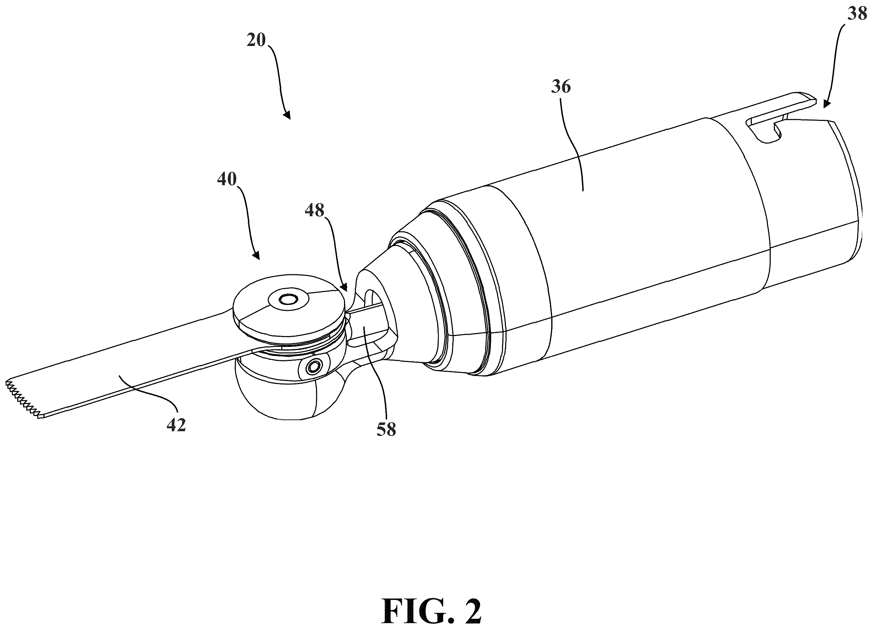

As best shown in FIGS. 1B-3, the sagittal saw attachment 20 includes a housing 36. The housing 36 of the sagittal saw attachment 20 may be configured to be operatively attached to the rotary surgical handpiece 24. For example, the housing 36 may define engagement detents 38 configured to engage corresponding engagement projections (not shown) defined by the rotary surgical handpiece 24. Of course, other configurations for operatively attaching the housing 36 of the sagittal saw attachment 20 to the rotary surgical handpiece 24 are contemplated. The sagittal saw attachment 20 also includes a blade mount 40 configured to releasably receive a surgical sagittal saw blade 42. The blade mount 40 is operatively attached to the housing 36 of the sagittal saw attachment 20 and supported for oscillating motion about a pivot axis 44 transverse to the output axis 32, and defines a lateral axis 46 transverse to the pivot axis 44. Beyond the structure described above, the configuration of the blade mount 40 for releasably receiving the surgical sagittal saw blade 42 is not limited for the purposes of this disclosure. One exemplary blade mount 40 can be found in U.S. Patent No. 7,833,241, which is incorporated by reference in its entirety herein.

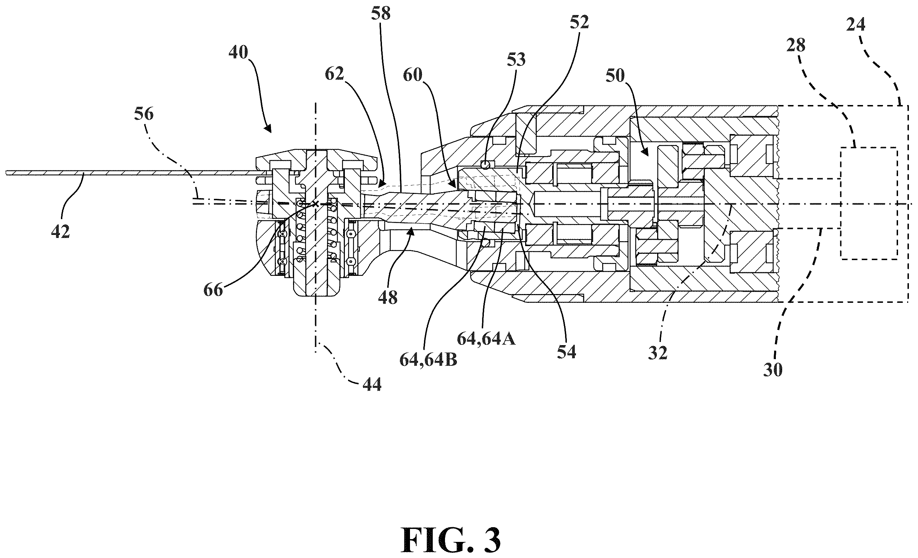

As best shown in FIGS. 3-6, the sagittal saw attachment 20 also includes a drive assembly 48 for converting rotary motion of the output shaft 30 of the rotary surgical handpiece 24 into oscillating motion of the blade mount 40 about the pivot axis 44. It should be appreciated that the drive assembly 48 of the present disclosure is not limited to applications within a sagittal saw attachment 20. In other words, the drive assembly 48 of the present disclosure may be employed in any suitable power tool for converting rotary motion of an output shaft into oscillating motion of a tool mount. According, the terms "tool mount" and "blade mount" are used interchangeably herein. In the configuration of FIGS. 3-6, the drive assembly 48 is connected to the blade mount 40 and is configured to be operatively attached to the output shaft 30 of the rotary surgical handpiece 24 to convert rotary motion of the output shaft 30 into oscillating motion of the blade mount 40 about the pivot axis 44.

With continued reference to FIGS. 3-6, the drive assembly 48 includes an off-center rotation member 52 configured to be operatively attached to the output shaft 30 of the rotary surgical handpiece 24 such that the off-center rotation member 52 rotates about the output axis 32 in response to actuation of the motor 28 of the rotary surgical handpiece 24. It should be appreciated that intervening components, such as a reduction gearset 50 (shown in FIG. 3) may be disposed between the output shaft 30 and the off-center rotation member 52 for providing a desired amount of torque to the drive assembly 48. Additionally, in some configurations, the sagittal saw attachment 20 may further include a seal member 53 arranged to contact an outer surface the off-center rotation member 52 and an internal surface the housing 36 to prevent debris from entering further into the sagittal saw attachment 20 and/or the rotary surgical handpiece 24.

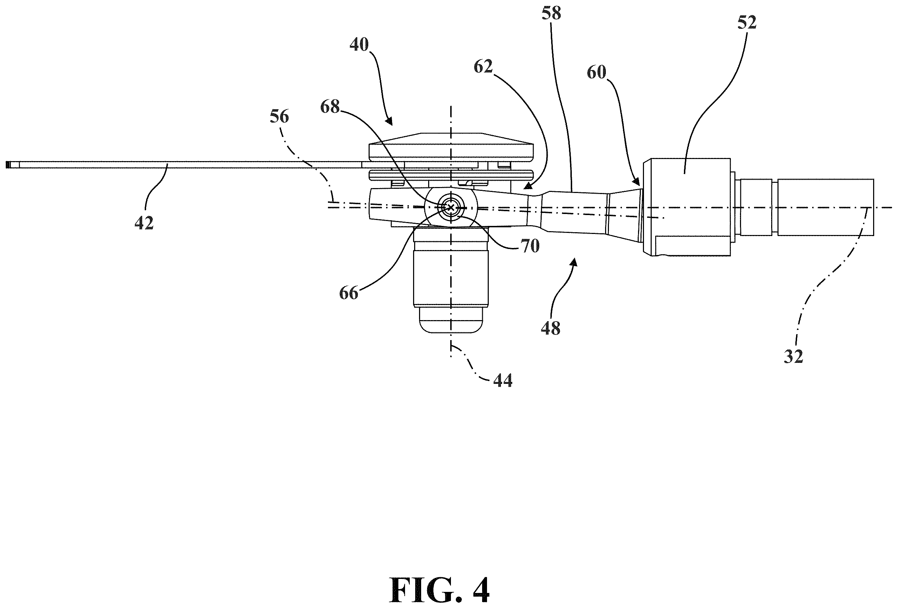

The off-center rotation member 52 also defines a coupling void 54. The coupling void 54 is radially spaced from the output axis 32 and concentric with a gimbal member axis 56 which intersects the output axis 32 and the pivot axis 44. As best shown in FIGS. 3 and 4, the gimbal member axis 56 is angularly offset from the output axis 32. For example, the gimbal member axis 56 may be offset from the output axis 32 by at least 1 degree, at least 2 degrees, at least 3 degrees, at least 5 degrees, at least 10 degrees, at least 15 degrees, at least 30 degrees, etc. All values between the exemplary angular offset values described above are expressly contemplated. The drive assembly 48 also includes a gimbal member 58 extending along the gimbal member axis 56 between a first end portion 60 and a second end portion 62. The first end portion 60 of the gimbal member 58 is supported within the coupling void 54 of the off-center rotation member 52 such that the gimbal member 58 is movable relative to the coupling void 54 in only one degree of freedom. Particularly, the first end portion 60 of the gimbal member 58 may be supported for rotation relative to the coupling void 54 about the gimbal member axis 56 such that the first end portion 60 revolves around the output axis 32 in response to actuation of the motor 28 (illustrated in phantom in FIG. 3). For example, the drive assembly 48 may further include one or more cylindrical bearing members 64 disposed in the coupling void 54 for supporting the first end portion 60 of the gimbal member 58 relative to the coupling void 54 such that the gimbal member 58 is movable relative to the coupling void 54 in only one degree of freedom (i.e., about the gimbal member axis 56). In some implementations, such as shown in FIGS. 3-6, the one or more cylindrical bearing members 64 may include a first cylindrical bearing member 64A and a second cylindrical bearing member 64B spaced from the first cylindrical bearing member 64A. For example, the first cylindrical bearing member 64A and the second cylindrical bearing member 64B may be arranged within the coupling void 54 and co-axial with the gimbal member axis 56. The cylindrical bearing member(s) 64 may include ball bearings, journal bearings, or the like.

The second end portion 62 of the gimbal member 58 is coupled to the blade mount 40 to oscillate the blade mount 40 about the pivot axis 44. In doing so, the second end portion 62 is configured to pivot relative to the lateral axis 46 as the gimbal member 58 oscillates the blade mount 40 about the pivot axis 44 in response to the first end portion 60 of the gimbal member 58 revolving around the output axis 32 in response to actuation of the motor 28. Accordingly, the blade mount 40 is configured to pivot about the pivot axis 44 along an arc. The arc may generally correspond to twice the angular offset of the gimbal member axis 56 from the output axis 32. For example, the arc may be at least 2 degrees, at least 4 degrees, at least 6 degrees, at least 20 degrees, at least 30 degrees, etc. All values between the exemplary arc values described above are expressly contemplated.

Several configurations of coupling the second end portion 62 of the gimbal member 58 to the blade mount 40 are contemplated. For example, in one configuration, the second end portion 62 of the gimbal member 58 may be coupled to the blade mount 40 via at least one pin 68 disposed along the lateral axis 46 such that the second end portion 62 of the gimbal member 58 oscillates the blade mount 40 about the pivot axis 44 in response to the first end portion 60 of the gimbal member 58 revolving around the output axis 32 in response to actuation of the motor 28. In these configurations, the drive assembly 48 may further include at least one pin bearing member 70 disposed between the at least one pin 68 and the blade mount 40 to permit rotation of the second end portion 62 of the gimbal member 58 relative to the blade mount 40 about the lateral axis 46. Here, the at least one pin bearing member 70 permits rotation of the second end portion 62 of the gimbal member 58 relative to the blade mount 40 about the lateral axis 46. The at least one pin bearing member 70 may include ball bearings, journal bearings, or the like.

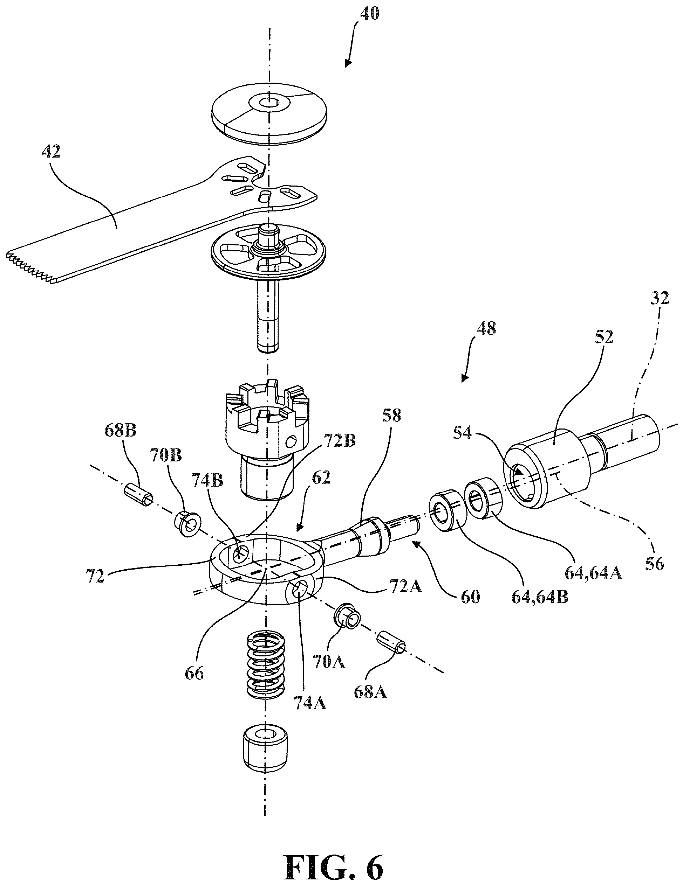

In another example of coupling the second end portion 62 of the gimbal member 58 to the blade mount 40, as best shown in FIG. 6, the second end portion 62 of the gimbal member 58 may define a rim portion 72 that at least partially surrounds the blade mount 40. Here, the first lateral side 72A of the rim portion 72 may define a first pin void 74A extending along the lateral axis 46, and a second lateral side 72B of the rim portion 72, opposite the first lateral side 72A, defines a second pin void 74B extending along the lateral axis 46. In this example, the drive assembly 48 may further include a first pin member 68A disposed in the first pin void 74A and a second pin member 68B disposed in the second pin void 74B. Here, each of the first pin member 68A and the second pin member 68B may be operatively attached to the blade mount 40 such that the second end portion 62 of the gimbal member 58 oscillates the blade mount 40 about the pivot axis 44 in in response to the first end portion 60 of the gimbal member 58 revolving around the output axis 32 in response to actuation of the motor 28. Additionally, the drive assembly 48 may further include a first pin bearing member 70A disposed between the first pin member 68A and the first pin void 74A and a second pin bearing member 70B disposed between the second pin member 68B and the second pin void 74B. Here, each of the first pin bearing member 70A and the second pin bearing member 70B permit rotation of the second end portion 62 of the gimbal member 58 relative to the blade mount 40 about the lateral axis 46. The first pin bearing member 70A and the second pin bearing member 70B may include ball bearings, journal bearings, or the like.

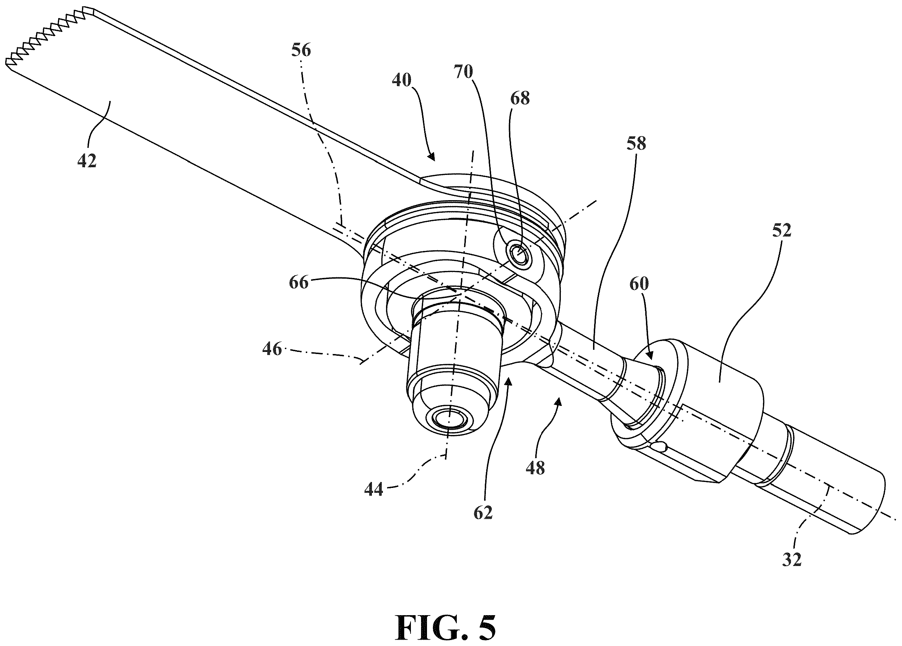

As best shown in FIGS. 5 and 6, the drive assembly 48 of the subject disclosure provides the advantage of having the output axis 32, the pivot axis 44, the lateral axis 46, and the gimbal member axis 56 meet at a common intersection point 66. As a result, the stress placed on the components of the drive assembly 48 and the rotating mass (and thus, the vibration during operation) of the drive assembly 48 is meaningfully reduced compared to conventional drive assemblies for converting rotary motion into oscillating motion. Furthermore, limiting the movement of the first end portion 60 relative to the coupling void 54 to only one degree of freedom provides meaningful improvements in component wear compared to conventional drive assembly configurations because conventional drive assembly configurations employ spherical bearings (which provide two degrees of freedom) which are prone to causing additional wear.

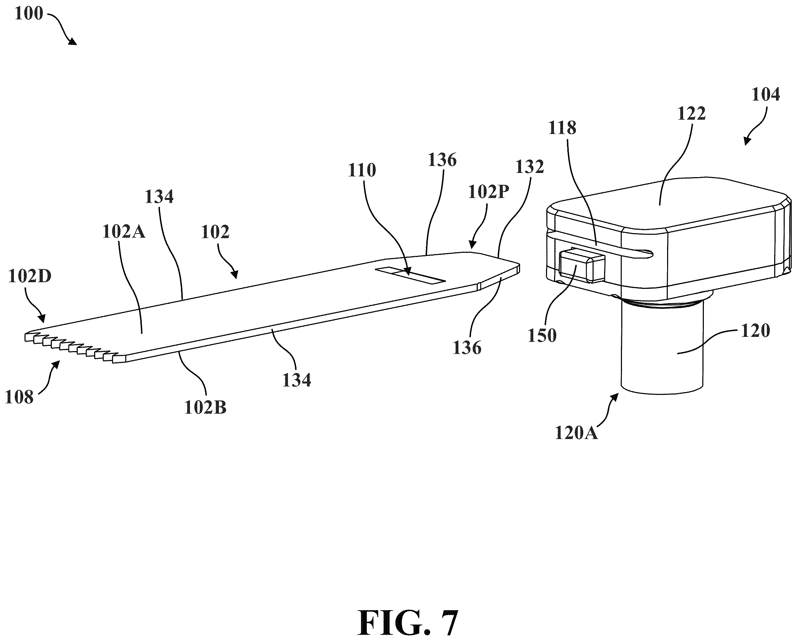

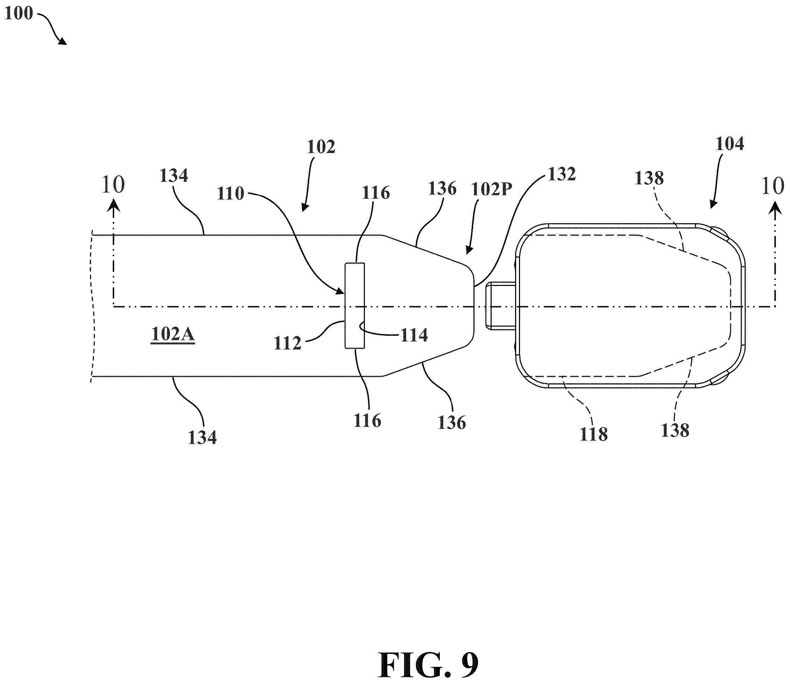

Referring to FIGS. 7-11C, the present disclosure is also directed to a sagittal saw system 100 including a saw blade 102, a blade mount 104 for releasably receiving the saw blade 102, and a retention member 106 for retaining the saw blade 102 relative to the blade mount 104. Referring first to FIG. 7, the saw blade 102 extends between a distal portion 102D and a proximal portion 102P. The saw blade 102 may include a first surface 102A (e.g. a top surface) and an opposing second surface 102B (e.g. a bottom surface). The distal portion 102D may define a plurality of teeth 108 configured to cut tissue, bone, or the like. The proximal portion 102P of the saw blade 102 defines an engagement slot 110. The engagement slot 110 may extend between the first surface 102A and the second surface 102B of the saw blade 102. In other words, the engagement slot 110 may extend through the entire thickness of the saw blade 102. However, it is contemplated that the engagement slot 110 may not fully extend through the saw blade 102. As best shown in FIG. 9, the engagement slot 110 may be bounded by a distal wall 112, a proximal wall 114, and opposing lateral walls 116 extending between the distal wall 112 and the proximal wall 114.

As described in further detail below, the blade mount 104 defines a blade channel 118 sized to at least partially receive the saw blade 102 (particularly, the proximal portion 102P of the saw blade 102), and the retention member 106 is supported by the blade mount 104 for movement between a plurality of retention member positions. The plurality of retention member positions includes an engaged position 106E where the retention member 106 abuts the engagement slot 110 of the saw blade 102 to retain the saw blade 102 within the blade channel 118. The plurality of retention member positions also includes a disengaged position 106D where the retention member 106 is spaced from the engagement slot 110 of the saw blade 102 to permit a user to translate the saw blade 102 distally to remove the saw blade 102 from the blade channel 118.

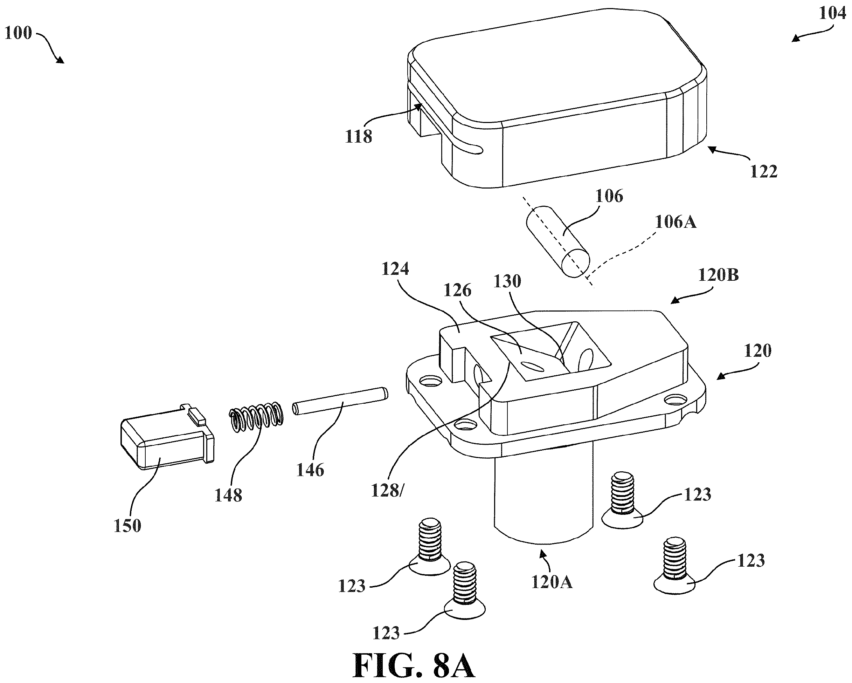

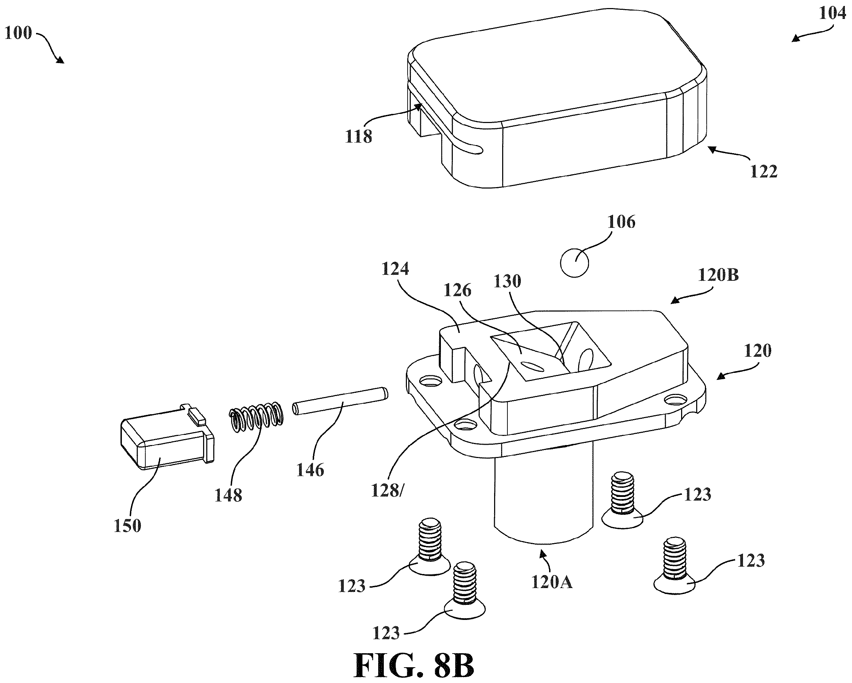

FIGS. 8A and 8B are exploded representations of the blade mount 104 and the retention member 106. The blade mount 104 may include a stem member 120 and a cap 122. The stem member 120 may extend between a first end portion 120A and a second end portion 120B. The first end portion 120A of the stem member 120 may be configured to be operatively attached to a motor of a rotary surgical handpiece (e.g. via the drive assembly 48) such that the motor may oscillate the blade mount 104 and the saw blade 102 to cut tissue, bone, or the like. The second end portion 120B of the stem member 120 may define a first stem surface 124 and a ramp portion 126. In some configurations, the ramp portion 126 extends between a first ramp edge 128 and a second ramp edge 130. The ramp portion 126 may be arranged at an acute angle relative to the first stem surface 124 such that the first ramp edge 128 is arranged distal to the second ramp edge 130. The retention member 106 may be arranged for movement along the ramp portion 126 between the plurality of retention member positions. In one configuration, such as shown in FIG. 8A, the retention member 106 is a cylindrical retention member 106, and a central axis 106A of the cylindrical retention member 106 may be parallel with the first ramp edge 128 and the second ramp edge 130 as the cylindrical retention member 106 moves between the plurality of retention member positions. In another configuration, such as shown in FIG. 8B, the retention member is a spherical retention member 106 configured for movement along the ramp portion 126 between the plurality of retention member positions. The ramp portion 126 may be laterally sized to laterally constrain the retention member 106. Other shapes of the retention member 106 are contemplated.

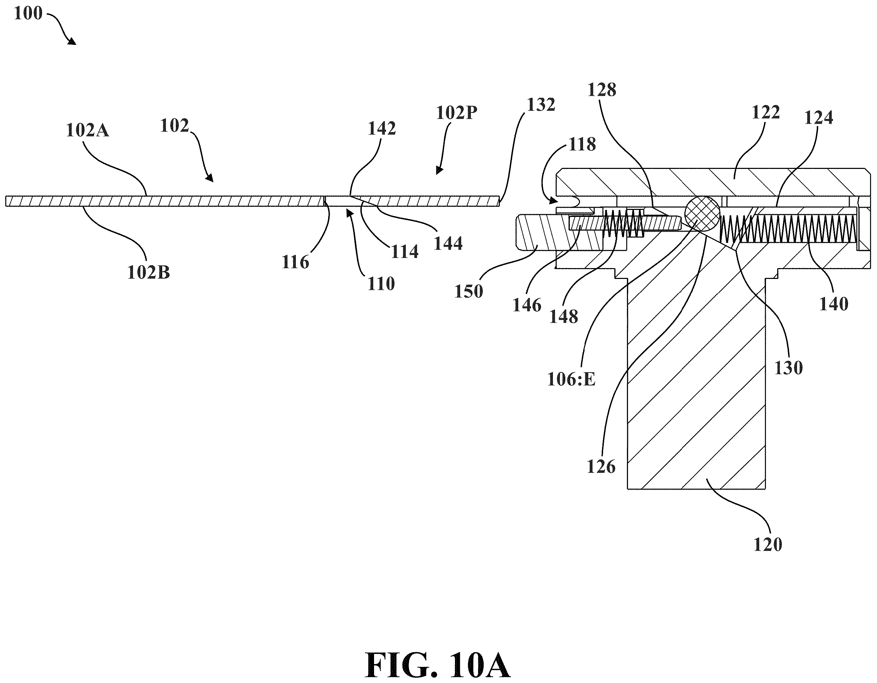

The cap 122 may be coupled to the second end portion 120B of the stem member 120 (e.g. via fasteners 123) such that the cap 122 and the first stem surface 124 of the stem member 120 cumulatively define the blade channel 118 (best shown in the cross-sectional representations of FIGS. 10A-11C). Additionally, as best shown in FIG. 9, in some examples, the proximal portion 102P of the saw blade 102 may include a proximal blade wall 132 and two lateral blade walls 134. The proximal portion 102P of the saw blade 102 may also define a tapered blade wall 136 between the proximal blade wall 132 and each of the lateral blade walls 134. In these examples, as best shown in phantom in FIG. 9, the blade channel 118 may define tapered channel walls 138 corresponding to the tapered blade walls 136. The tapered channel walls 138 of the blade channel 118 may be configured to abut the tapered blade walls 136 of the saw blade 102 to align the saw blade 102 relative to the blade mount 104.

FIGS. 10A-10E illustrate a sequence of inserting the saw blade 102 into the blade channel 118 such that the retention member 106 retains the saw blade 102 within the blade channel 118. FIG. 10A shows the saw blade 102 spaced from the blade mount 104. It should be appreciated that the sagittal saw system 100 may further include a biasing member 140 supported by the blade mount 104 and operatively attached to the retention member 106 to urge the retention member 106 to the engaged position 106E. Here, the biasing member is disposed within the stem member 120, but other configurations are contemplated. Accordingly, in the present configuration, where the saw blade 102 is spaced from the blade mount 104, the retention member 106 is in the engaged position 106E.

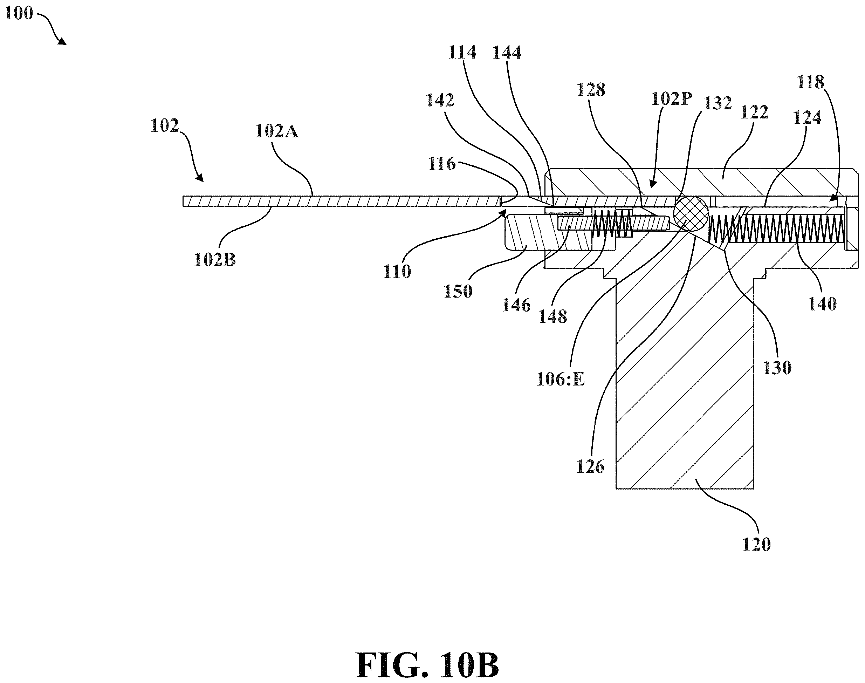

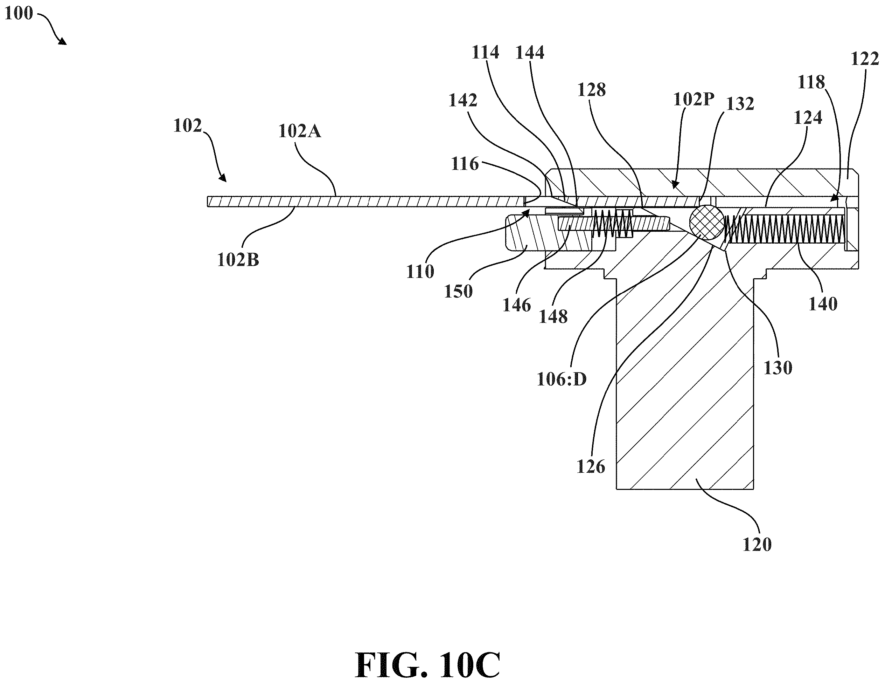

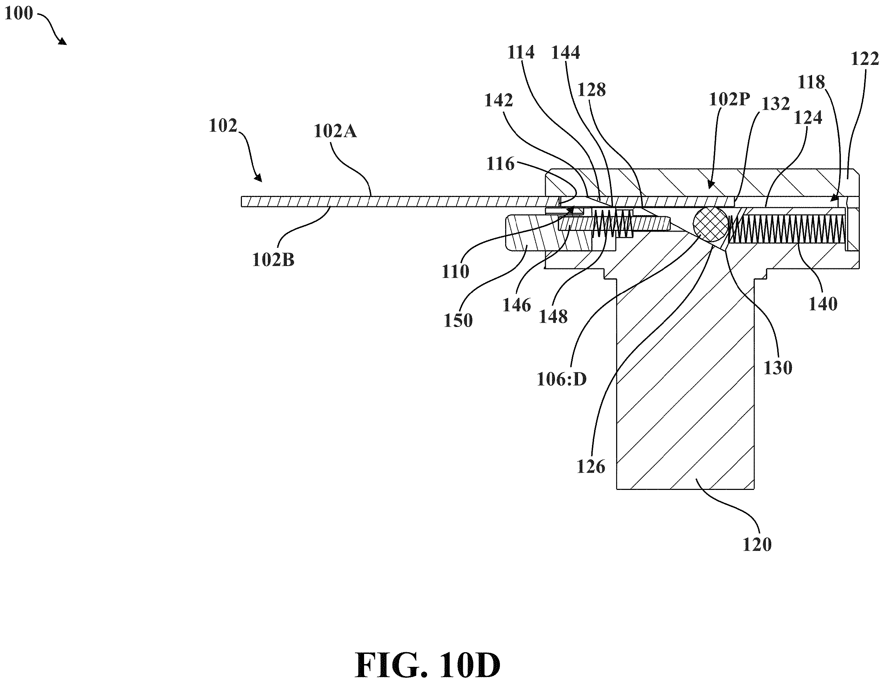

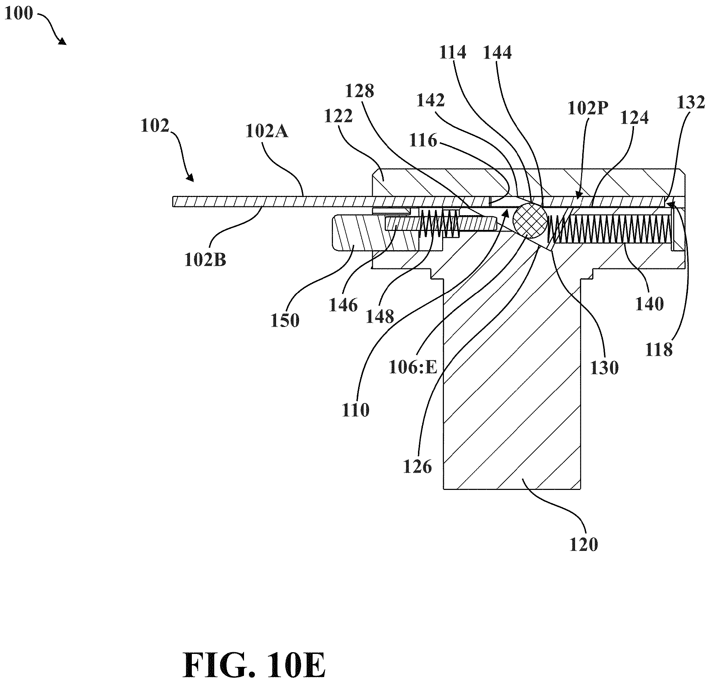

Referring now to the sequence between FIGS. 10B and 10C, in some configurations, the proximal blade wall 132 may be configured to abut the retention member 106 in response to insertion of the saw blade 102 into the blade channel such that the proximal blade wall 132 moves the retention member 106 to the disengaged position 106D as the saw blade 102 is initially inserted in the blade channel 118. Accordingly, referring to FIG. 10D, with the retention member 106 displaced to the disengage position 106D, the saw blade 102 may be further inserted into the blade channel 118. Advantageously, this configuration allows the saw blade 102 to be inserted into the blade channel 118 without any other user engagement with the blade mount 104 (such as unlocking a lock mechanism, pushing a button, etc.); instead, a user may simply insert the saw blade 102 into the blade channel 118 of the blade mount 104. Referring lastly to FIG. 10E, the biasing member 140 may be configured to urge the retention member 106 to the engaged position 106E in response to the saw blade 102 being fully inserted into the blade channel 118 to retain the saw blade 102 within the blade channel 118. In other words, once the saw blade 102 is fully inserted into the blade channel 118, the retention member 106 is aligned with the engagement slot 110 of the saw blade 102, and the biasing member 140 urges the retention member 106 to the engaged position 106E such that the retention member 106 abuts the engagement slot 110 to retain the saw blade 102 within the blade channel 118.

With continued reference to FIG. 10E, in the engaged position 106E, the retention member 106 may be configured to abut the proximal wall 114 of the engagement slot 110 of the saw blade 102 to retain the saw blade 102 within the blade channel 118. In some examples, the proximal wall 114 of the engagement slot 110 extends between a first edge 142 and a second edge 144. The proximal wall 114 may be arranged at an obtuse angle relative to the second surface 102B of the saw blade 102 such that the first edge 142 is closer to the distal portion 102D of the saw blade 102 than the second edge 144. Advantageously, by arranging the proximal wall 114 of the engagement slot 110 at an obtuse angle, any effort to remove the saw blade 102 from the blade channel 118 (i.e., pull the saw blade 102 distally) will result in the retention member 106 urging the first surface 102A of the saw blade 102 upward (e.g. against the cap 122) such that the frictional force of the first surface 102A of the saw blade 102 against the blade channel 118 retains the saw blade 102 within the blade channel 118.

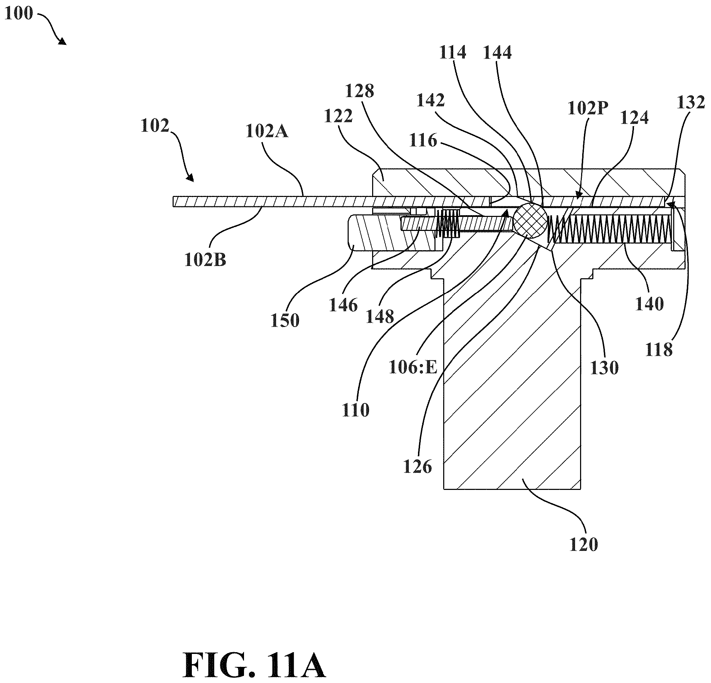

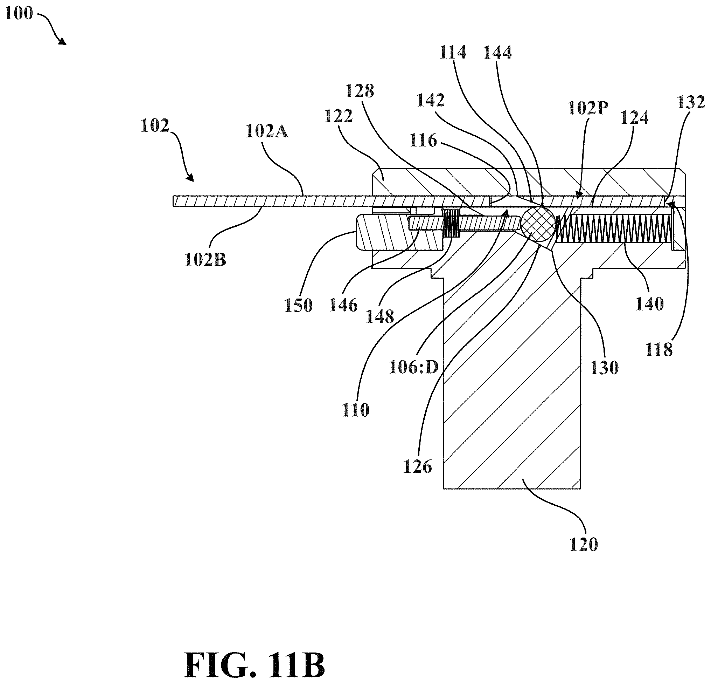

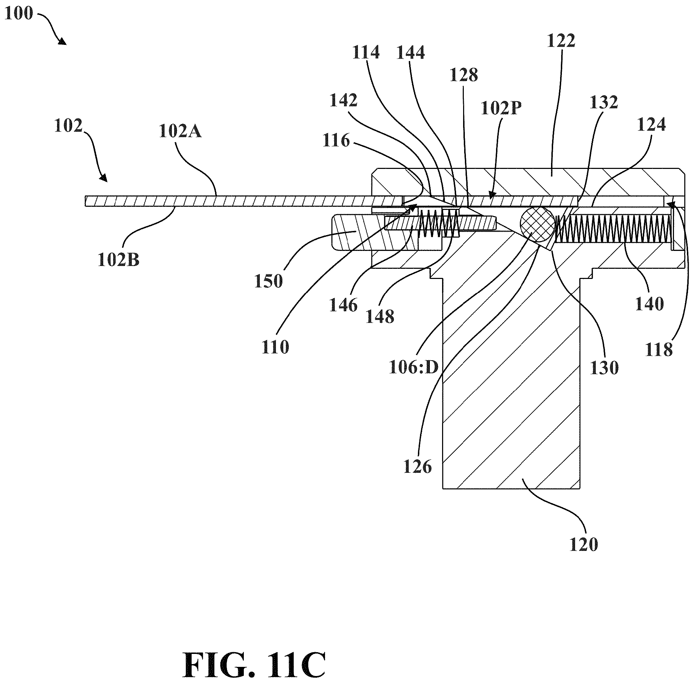

In some examples, the sagittal saw system 100 may also further include a release member 146 operatively attached to the blade mount 104 (e.g. supported at least partially within the stem member 120) for moving the retention member 106 from the engaged position 106E to the disengaged position 106D. The release member 146 may be arranged for movement relative to the blade mount 104 such that the release member 146 is configured to abut the retention member 106 to move the retention member 106 from the engaged position 106E to the disengaged position 106D in response to user engagement. FIGS. 11A-11C illustrate a sequence of operating the release member 146. FIG. 11A shows the release member 146 in the absence of user engagement. In the illustrated configuration, the sagittal saw system 100 also includes a release biasing member 148 arranged to urge the release member 146 away from the retention member 106 such that the release member 146 is spaced from the retention member 106 in the absence of user engagement. FIG. 11B illustrates initial user engagement with the release member 146 such that the release member 146 abuts the retention member 106. FIG. 11C shows continued user engagement with the release member 146 such that the release member 146 moves the retention member 106 from the engaged position 106E to the disengaged position 106D. Here, the retention member 106 is spaced from the engagement slot 110 of the saw blade 102 such that the saw blade 102 may be translated distally to remove the saw blade 102 from the blade channel 118. In some examples, a button 150 is operatively attached to the releaser member 146 and arranged for user engagement to move the release member 146. Other configurations of the release member 146 for moving the retention member 106 from the engaged position 106E to the disengaged position 106D are contemplated.

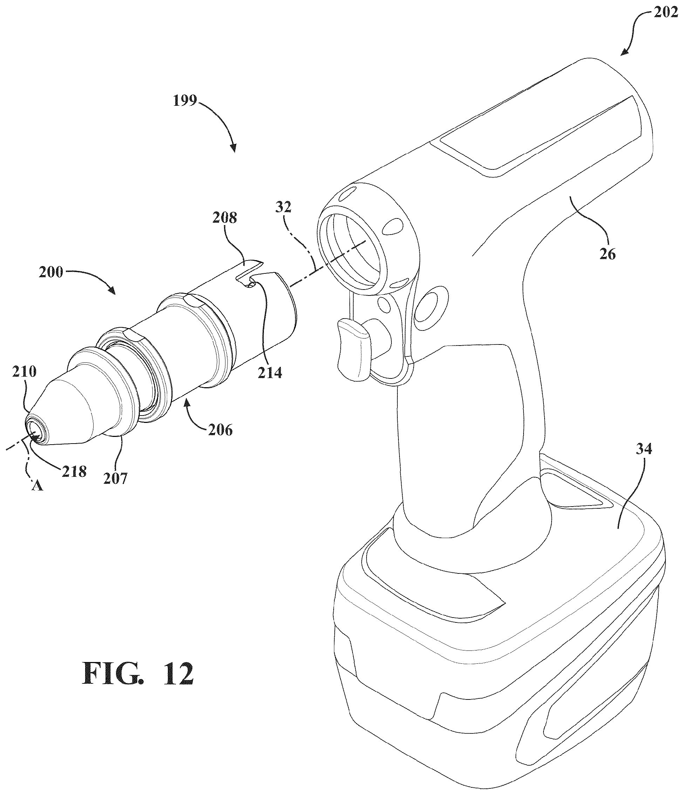

As shown throughout Figures 12-17 and 21, the present disclosure also provides a surgical system 199. The surgical system includes a surgical attachment 200 for coupling to a surgical driver 202 and a surgical accessory tool 204. In other words, the surgical attachment 200 couples the surgical driver 202 with the surgical accessory tool 204. The surgical driver 202 is not particularly limited. In some implementations, the surgical attachment 200 may also be referred to as a surgical assembly 200. In some implementations, the surgical attachment 200 and the surgical driver 202 may collectively form the surgical assembly 200, such that the surgical attachment 200 and the surgical driver 202 are integral. Similar to the rotary surgical handpiece 24 described above, suitable surgical drivers 202 include a body 26 and a motor 28 disposed within the body 26. The motor 28 includes an output shaft 30 configured to be rotated about an output axis (A) 32 in response to actuation of the motor 28. Beyond the structure described above, the configuration of the surgical driver 202 is not particularly limited for the purposes of this disclosure. For example, the surgical driver 202 may be the rotary surgical handpiece 24 describe above or the surgical drivers 202 described in U.S. Pat. Pub. No. 2007/0021766 or U.S. Patent No. 10,537,339, which are again expressly incorporated by reference in their entirety herein.

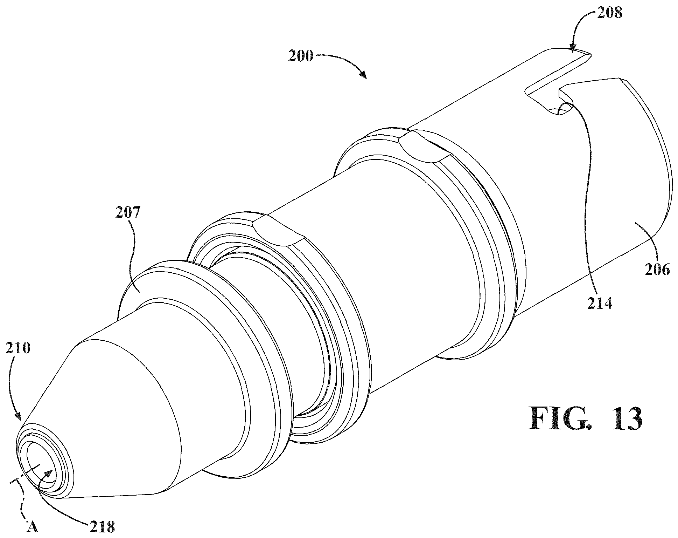

As best shown in Figures 12 and 13, the surgical attachment 200 may include a housing 206 and extend from a first end portion 208 for coupling to the surgical driver 202 and a second end portion 210 opposite the first end portion 208. The first end portion 208 may also be referred to as the proximal end portion and the second end portion 210 may be referred to as the distal end portion, with the distal end portion being relatively closer to the patient than the operator (typically a medical professional) of the surgical attachment 200 and the proximal end portion being relatively closer to the operator. The first end portion 208 may comprise a first coupler and the driver 202 may comprise a second coupler (not shown). The first and second couplers may facilitate removably attachment of the surgical attachment 200 to the surgical driver 202.

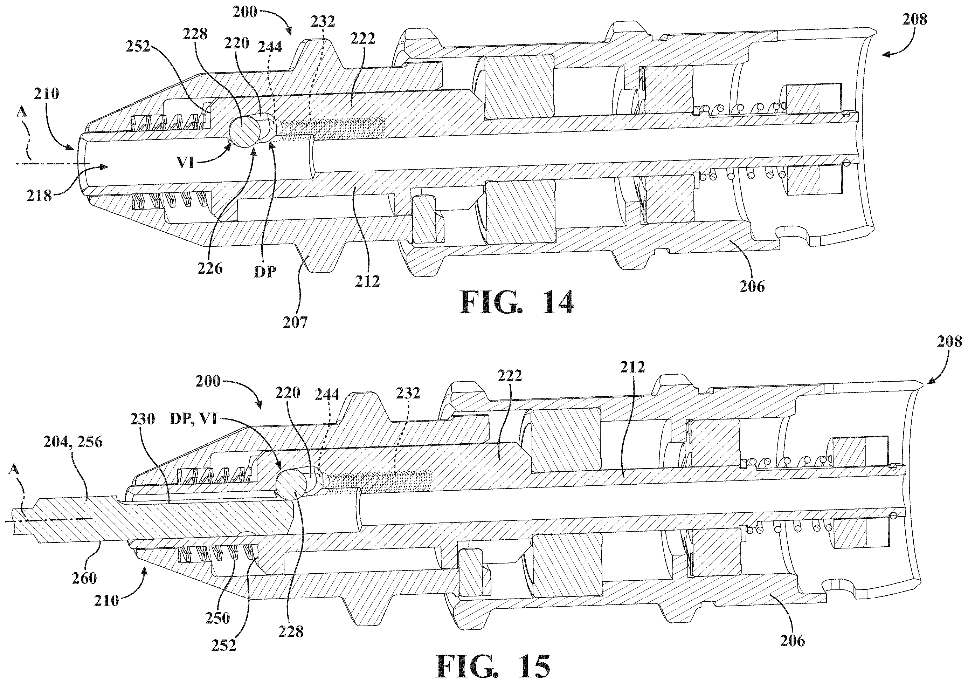

With reference to Figures 14-21, the surgical attachment 200 also includes a driving member 212 at least partially disposed within the housing 206, with the driving member 212 being rotatable about an axis (A) relative to the housing 206 and configured to transfer torque received from the surgical driver 202 to the surgical accessory tool 204. In some implementations, the driving member 212 may be monolithic. In other words, the driving member may be of unitary construction. Such an implementation may reduce the number of components and provide for easier assembly of the surgical assembly 200. The housing 206 of the surgical attachment 200 may couple to the surgical driver 202 by any suitable means, provided that the coupling is operative to transfer torque from the surgical driver 202 to the driving member 212 of the surgical attachment 200. For example, as best shown in Figure 13, the housing 206 may include engagement detents 214 configured to engage corresponding engagement projections (not shown) defined by the surgical driver 202.

Referring specifically to the driving member 212, those skilled the art may also refer to the driving member 212 as a drive shaft. The driving member 212 may define a receiving bore 218 extending along the axis (A) for receiving the surgical accessory tool 204. The driving member 212 also defines a first opening 220 extending transverse to and partially into the receiving bore 218. In other words, the driving member 212 includes a first opening 220 for providing access to the receiving bore 218.

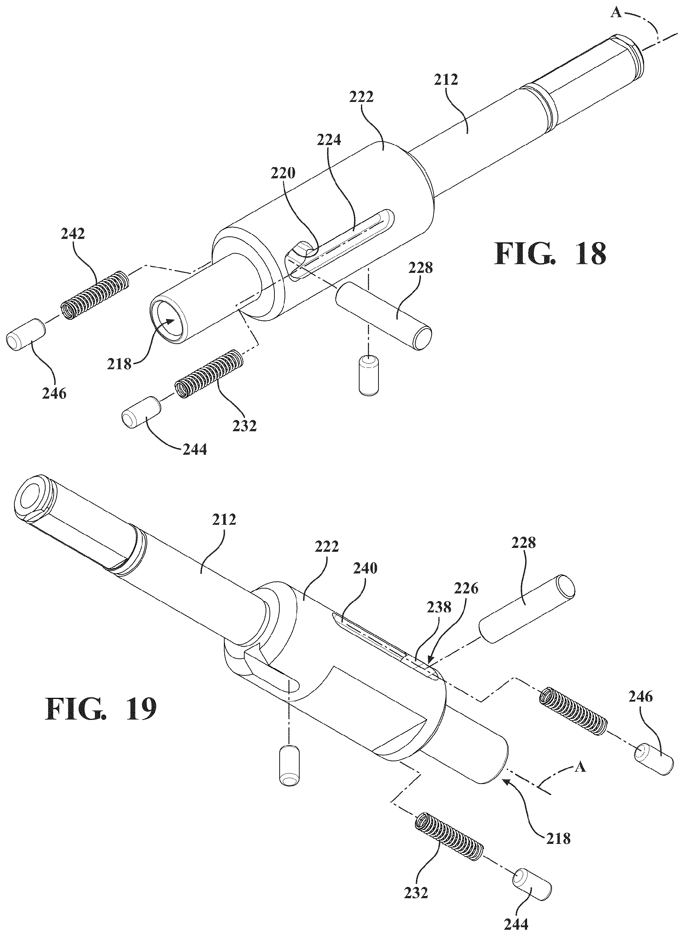

Referring now to Figures 14-20, the surgical attachment 200 may also include a shoulder 222 extending from and disposed around the driving member 212. The shoulder 222 may comprise or define various features described below. It is contemplated that in implementations where the driving member 212 does not include a shoulder 222, the driving member 212 may still include such features. The shoulder 222 of the driving member 212 defines a first cavity 224 that is in communication with the first opening 220 of the driving member 212. As shown in Figures 18-20, the first cavity 224 may resemble a canal defined in the shoulder 222 of the driving member 212, which merges with the first opening 220 defined in the driving member 212. As described further below, the first opening 220 and the first cavity 224 cooperate to support a lock assembly 226 for securing the surgical accessory tool 204 to the surgical attachment 200.

The lock assembly 226 includes a retention member 228 partially disposed in the first opening 220 of the driving member 212 for movement between a plurality of retention member 228 positions. Typically, the retention member 228 is cylindrically shaped such that the retention member 228 may be referred to as a cylindrical retention member 228. It is contemplated that the retention member 228 may comprise an alternative shape. Typically, at least a portion of the retention member 228 comprises a rounded surface. The plurality of retention member 228 positions includes a disengaged position (DP) where a first volume (V1) of the retention member 228 is disposed in the receiving bore 218 of the driving member 212. The disengaged position (DP) may be referred to as a first position. The plurality of retention member 228 positions also include an engaged position (EP) where a second volume (V2) of the retention member 228 is disposed in the receiving bore 218 of the driving member 212, with the second volume (V2) being less than the first volume (V1). The engaged position (EP) may be referred to as a second position. Said differently, when the retention member 228 is in the disengaged position (DP), a relatively larger amount of the retention member 228 is disposed in the receiving bore 218 and when the retention member 228 is in the engaged position (EP), a relatively lesser amount of the retention member 228 is disposed in the receiving bore 218. As described further below, the retention member 228 is in the disengaged position (DP) when the lock assembly 226 is not engaged with the surgical accessory tool 204. Conversely, the retention member 228 is in the engaged position (EP) when the lock assembly 226 is engaged with a flat region 230 of the surgical accessory tool 204.

The lock assembly 226 may further include a first retention biasing member 232 at least partially disposed in the first cavity 224 of the shoulder 222 of the driving member 212. Although not required, as shown in Figures 18-20, the first cavity 224 of the shoulder 222 containing the first biasing member 232 generally extends substantially parallel to the axis (A) of the driving member 212. Within the context of this disclosure, substantially parallel means +/- 5° from parallel. The first biasing member 232 may be any type of biasing member, provided that the selected biasing member is configured to urge the retention member 228 towards the disengaged position (DP). The retention member 228 in the disengaged position (DP) is disposed distally relative to the retention member 228 in the engaged position (DP). For example, the biasing member may be a spring or a wave spring. In the disengaged position (DP), the force exerted from the biasing member is the only force acting upon the retention member 228, which urges a relatively greater amount of the retention member 228 into the receiving bore 218 of the driving member 212. In the engaged position (EP), the flat region 230 of the surgical accessory tool 204 also exerts a force on the retention member 228, which urges the retention member 228 further into the first opening 220 and consequently reduces the volume of the retention member 228 within the receiving bore 218.

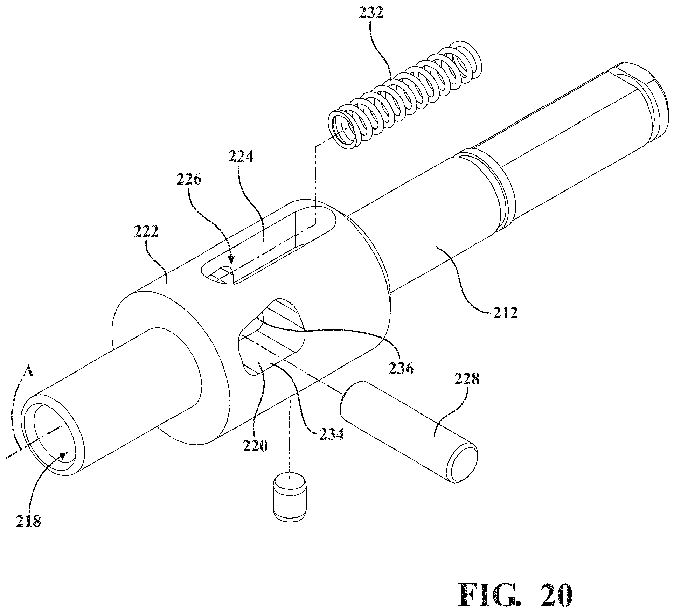

As shown in Figure 20, the driving member 212 may include a bottom surface 234 at least partially defining the first opening 220. The driving member 212 may also include a top planar surface 236, alternatively referred to as a ceiling 236, to further define the first opening 220. As shown in Figure 20, the top planar surface 236 that defines the first opening 220 may be angled relatively upward from a perspective beginning from the second end portion 210 (distal end portion) of the surgical attachment 200 extending towards the first end portion 208 (proximal end portion). Said differently, the top planar surface may face the axis (A) and be disposed at an angle between one and thirty degrees (between 1° and 30°) relative to the axis (A). In some implementations, the angle is between five and fifteen degrees (between 5° and 15°). A distance between the bottom surface 234 of the first opening 220 and the axis (A) extending along the driving member 212 may decrease as the driving member 212 extends from the first end portion 208 toward the second end portion 210 adjacent the first opening 220. Alternatively, as shown in Figure 18, the relative size of the first opening 220 may decrease as the driving member 212 extends from the first end portion 208 (proximal end portion) towards the second end portion 210 (distal end portion) adjacent the opening 220. In this alternative configuration, the distance between the bottom of the first opening 220 and the ceiling 236 of the first opening 220 decreases as the driving member 212 extends from the first end portion 208 (proximal end portion) towards the second end portion 210 (distal end portion) adjacent the opening 220.

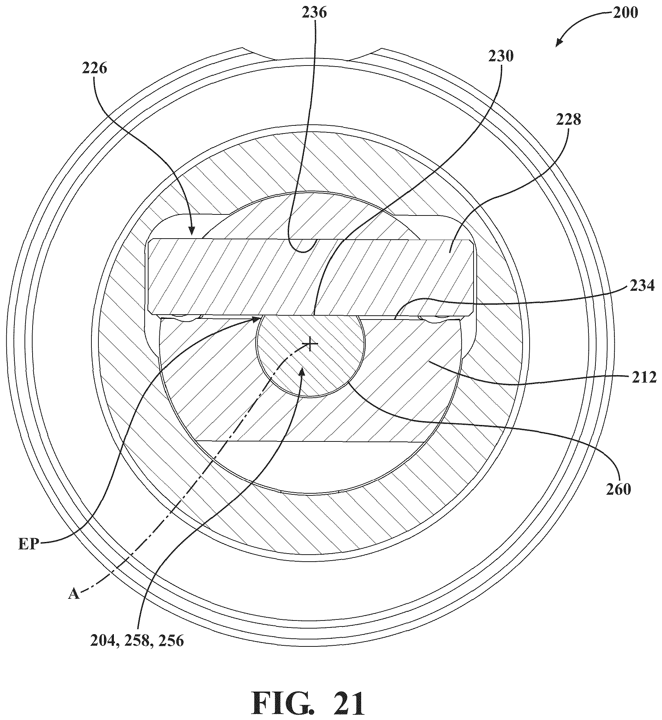

As shown in Figure 21, the retention member 228 in the engaged position (EP) may be spaced from the bottom surface 234 of the first opening 220 and in abutting contact with the top planar surface 236 and the flat region 230 of the surgical accessory tool. Collectively, the force transferred from the biasing member 232 to the retention member 228, the force exerted from the flat region 230 of the surgical accessory tool 204, and a contact force from the top planar surface 236 of the first opening 220 are configured to cooperate and axially secure the surgical accessory tool 204 within the receiving bore 218 of the driving member 212. The abutting engagement between the retention member 228 and the flat region 230 of the surgical accessory tool 204 is also sufficient to transfer torque between the surgical attachment 200 and the surgical accessory tool 204, and thus transfer the torque received from the surgical driver 202 to the surgical accessory tool 204. As described further below, this cooperative configuration allows medical professionals to couple the surgical accessory tool 204 to the surgical attachment 200 with a single hand by merely aligning the flat region 230 of the surgical accessory tool 204 with the retention member 228 and inserting the surgical accessory tool 204 into the receiving bore 218 of the driving member 212.

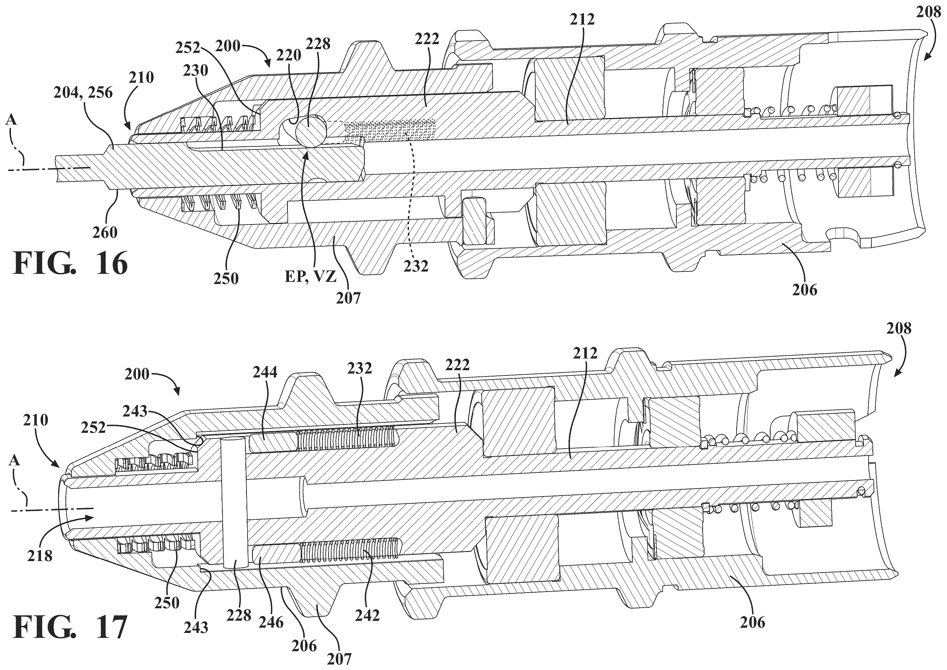

Although not required, as shown in Figure 19, the driving member 212 of the surgical attachment 200 may define a second opening 238, which is symmetrically oriented relative to the first opening 220 such that the retention member 228 is disposed in both the first and second openings 220, 238. In other words, the first and second openings 220, 238 are linearly aligned on opposite sides of the driving member 212 such that the first and second openings 220, 238 are configured to receive the retention member 228. As further shown in Figure 17, when the driving member 212 defines the second opening 238, the driving member 212 may also define a second cavity 240 to support a second retention biasing member 242. The second cavity 240 may be symmetrically oriented relative to the first cavity 224 across a reference plane that extends through the axis (A). For example, the reference plane may be the plane across which the section view of Figure 14 is taken. This configuration may advantageously be utilized to apply equal biasing forces on opposite sides of the retention member 228. This configuration may also include a first engagement pin 244 disposed partially within the first cavity 224 and partially within the first opening 220 between the first biasing member 232 and the retention member 228. The first engagement pin 244 may alternatively be referred to as a first ram 244. The first engagement pin 244 may be configured to transfer force from the first biasing member 232 to the retention member 228. The first engagement pin 244 may have various geometric configurations but is often cylindrically shaped, especially when the retention member 228 is cylindrical. Of course, a second engagement pin 246 may be similarly deployed between the second biasing member 242 and the retention member 228. Use of the first and/or second engagement pins 244, 246 advantageously ensures consistent force is applied from the first and/or second biasing members 232, 242 to the retention member 228.

Referring back to the housing 206, as best shown in Figures 13, 15, and 17, the surgical attachment 200 may include a release collar 207 at the second end portion 210 of the attachment 200. As the name implies, the release collar 207 is configured to be actuated by a medical professional to release the surgical accessory tool 204. The release collar 207 is biased distally via a release biasing member 250 sandwiched between the release collar 207 and an outer surface 252 of the shoulder 222 of the driving member 212. The outer surface 252 of the shoulder 222 may be adjacent and spaced distally from the retention member 228. Similar to the first and or second biasing members 232, 242, the release biasing member 250 may be any suitable biasing member, such as a spring or wave spring. To release the surgical accessory tool 204, the medical professional applies a force (i.e., an outside force) to the release collar 207 towards the first end portion 208 to overcome the biasing force of the first and/or second retention biasing members 232, 242 and the release biasing member 250 to guide a contact surface 243 of the release collar 207 to abutting contact with the retention member 228 and urge the retention member 228 proximally. The contact surface 243 of the release collar 207 is configured to avoid contact with the shoulder 222 of the driving member 212. The release biasing member 250 is disposed distal the retention member 228.

The present disclosure also provides a surgical handpiece system. The system includes the surgical driver 202 including a motor 28 for generating torque, the surgical attachment 200 described herein, and the surgical accessory tool 204. In certain implementations, the surgical accessory tool 204 AO drill bit 256.

With reference to Figures 20 and 21, the AO drill bit 256 includes a shaft 258 having the flat region 230. Generally, a cross-section of the AO drill bit 256 is circular with the exception of the flat region 230. As described above, when the flat region 230 of the AO drill bit 256 is inserted into the receiving bore 218 of the driving member 212, the flat region 230 contacts the retention member 228 and urges a portion of the retention member 228 to extend deeper into the first opening 220 of the driving member 212. However, if the AO drill bit 256 is received in a misaligned configuration within the receiving bore 218 such that a circular region 260 (i.e., a non-liner region) of the shaft 258 contacts the retention member 228 instead of the flat region 230, the additional mass of the shaft 258 about the circular region 259 (as opposed to the flat region 230) results in the retention member 228 contacting the ceiling 236 of the first opening 220 prior to the AO drill bit 256 being fully inserted into the receiving bore 218, which in turn prevents the AO drill bit 256 from coupling with the surgical attachment 200. In other words, the surgical handpiece system is configured to prevent the AO drill bit 256 from being secured in the receiving bore 218 when the flat region 230 of the shaft is misaligned with the retention member 228.

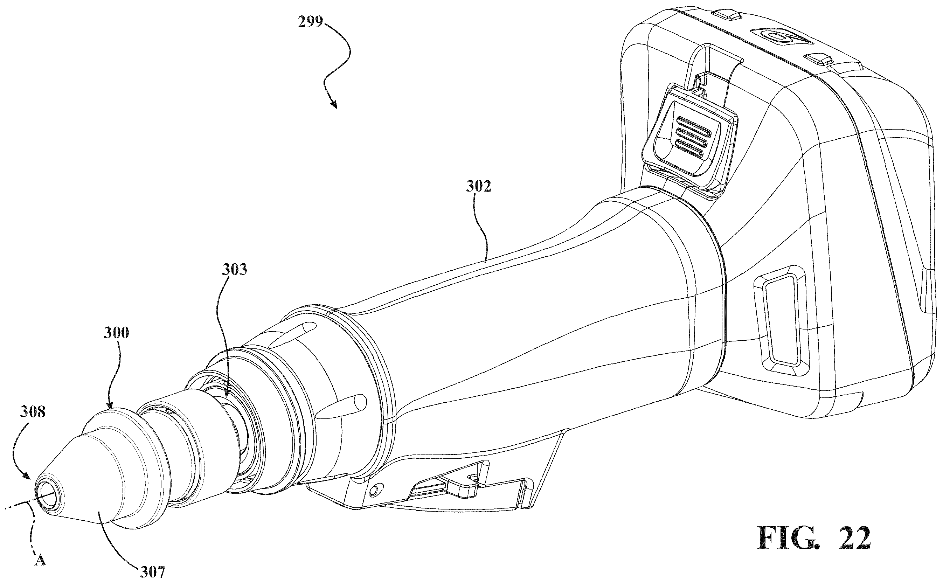

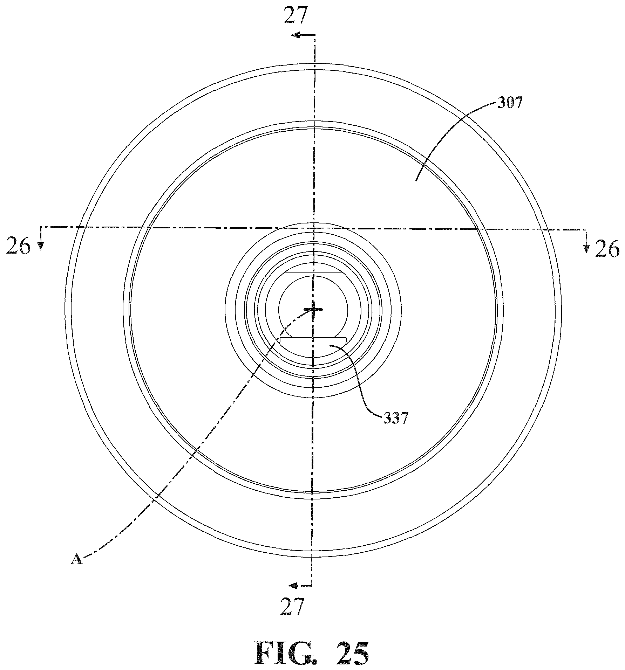

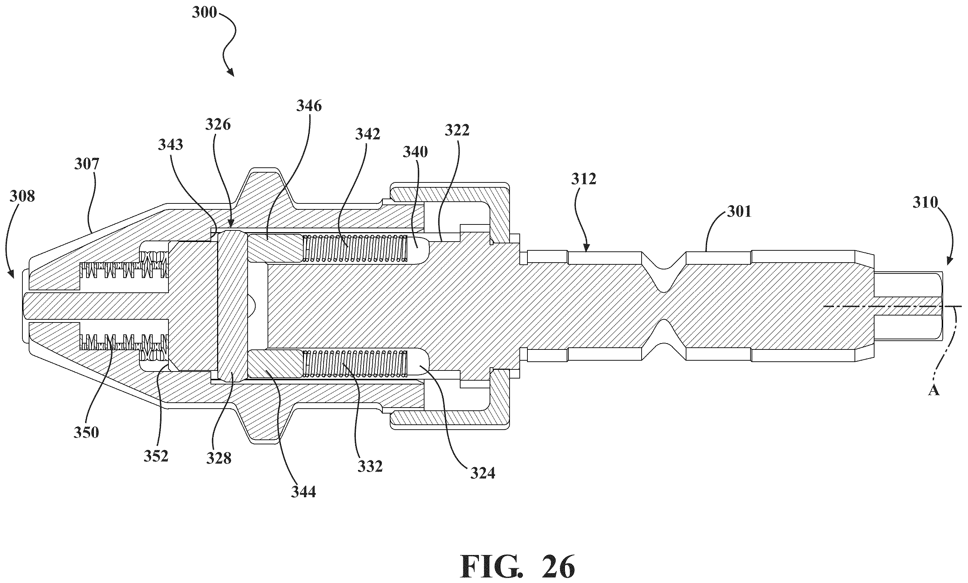

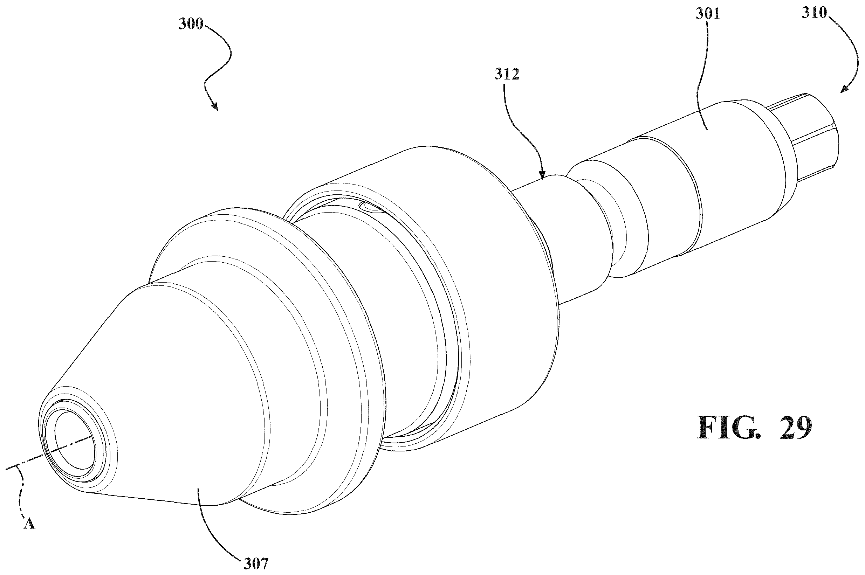

As shown throughout Figures 22-31, another implementation of a surgical system 299 is provided. The surgical system 299 includes a surgical attachment 300 for coupling to a surgical driver 302 and a surgical accessory tool 304. In other words, the surgical attachment 300 couples the surgical driver 302 with the surgical accessory tool 304. The surgical driver 302 is not particularly limited. In some implementations, the surgical attachment 300 may also be referred to as a surgical assembly 300. In some implementations, the surgical attachment 300 and the surgical driver 302 may collectively form the surgical assembly 300, such that the surgical attachment 300 and the surgical driver 302 are integral. Similar to the rotary surgical handpiece 24 described above, suitable surgical drivers 302 include a body 26 and a motor 28 disposed within the body 26. The motor 28 includes an output shaft 30 configured to be rotated about an output axis (A) 32 in response to actuation of the motor 28. Beyond the structure described above, the configuration of the surgical driver 302 is not particularly limited for the purposes of this disclosure. For example, the surgical driver 302 may be the rotary surgical handpiece 24 describe above or the surgical drivers 302 described in U.S. Pat. Pub. No. 2007/0021766 or U.S. Patent No. 10,537,339, which are again expressly incorporated by reference in their entirety herein. Another suitable example of a surgical driver 302 is described in PCT Pub. No. WO2024/035819, published on February 15, 2024, and herein incorporated by reference in its entirety.

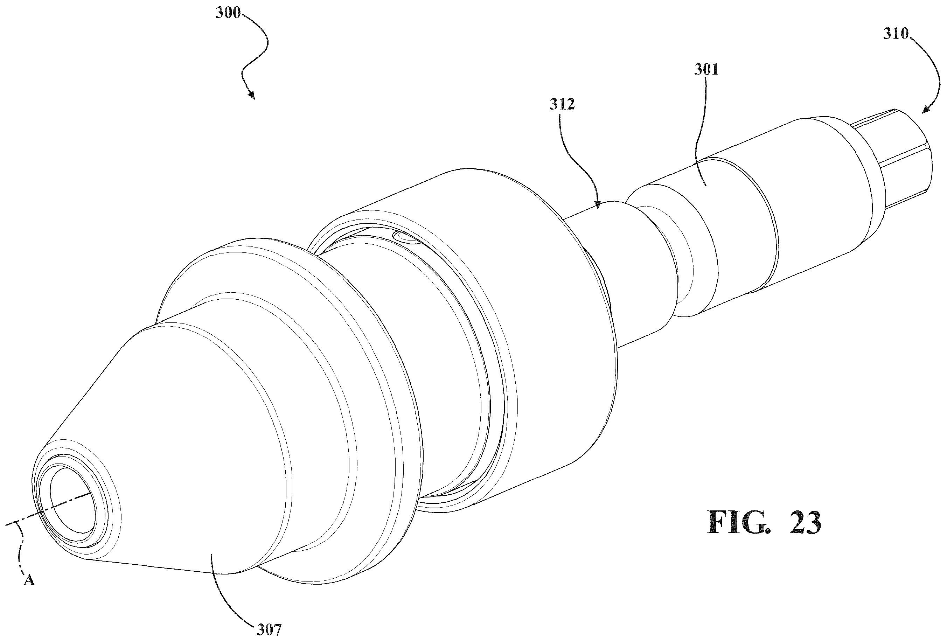

As shown in Figures 22 and 23, the surgical attachment 300 may include a driving member 312 being rotatable about an axis (A) and configured to transfer torque received from the surgical driver 302 to the surgical accessory tool 304. In contrast to the above-described surgical attachment 200, the surgical attachment 300 may not include a housing for coupling to the surgical driver 302. Instead, the driving member 312 may include an input shaft 301 that defines features for receiving torque from an output drive 303 of the surgical driver 302. Said differently, the surgical driver may have a first coupler 303 and the driving member may have a second coupler 301 adjacent a proximal end of the driving member 312. The second coupler 301 may be adapted for removable attachment to the first coupler 303 of the surgical driver 302. In this implementation of the surgical attachment 300, the entirety of the surgical attachment 300 may rotate in response to receiving torque from the surgical driver 302.

The input shaft 303 may also be referred to as a first end portion 308 of the surgical attachment 308 or a proximal end portion. The surgical attachment 300 may also include a second end portion 310 opposite the first end portion 308. The second end portion 310 may be referred to as the distal end portion, with the distal end portion being relatively closer to the patient than the operator (typically a medical professional) of the surgical attachment 300 and the proximal end portion being relatively closer to the operator. In some implementations, the driving member 312 may be monolithic. In other words, the driving member 312 may be of unitary construction. Such an implementation may reduce the number of components and provide for easier assembly of the surgical assembly 300.

Referring specifically to the driving member 312, those skilled the art may also refer to the driving member 312 as a drive shaft. The driving member 312 may define a receiving bore 318 extending along the axis (A) for receiving the surgical accessory tool 304. The driving member 312 also defines a first opening 320 extending transverse to and partially into the receiving bore 318. In other words, the driving member 312 includes a first opening 320 for providing access to the receiving bore 318.

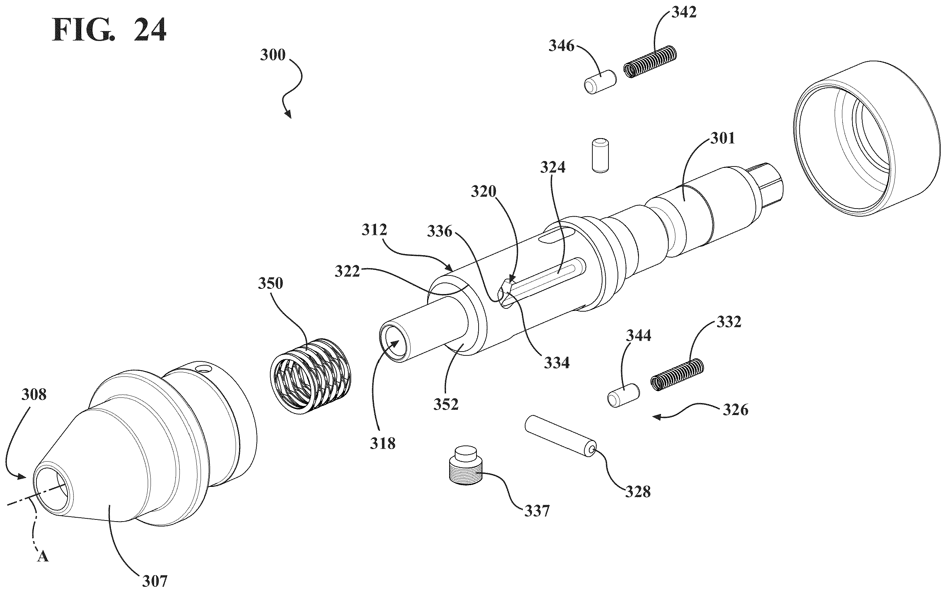

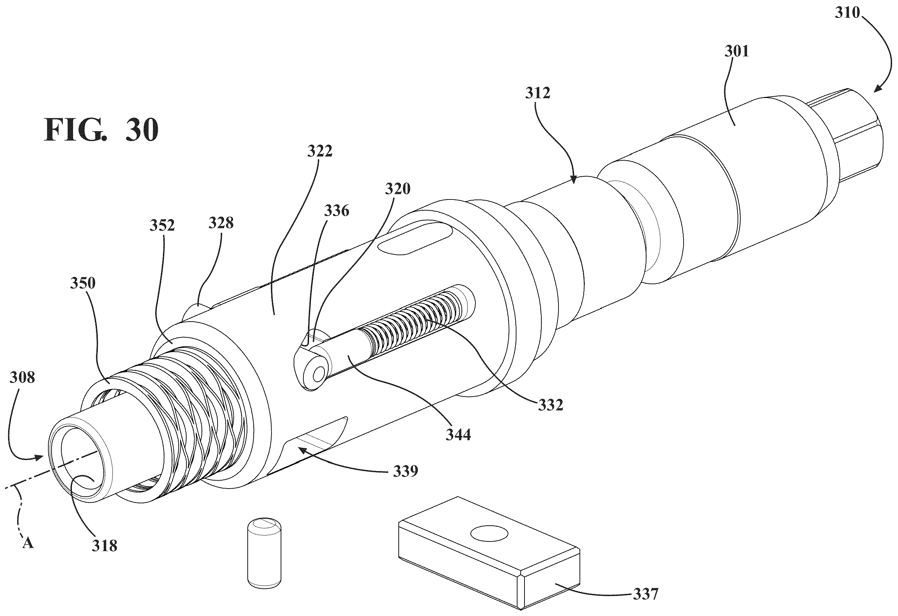

Referring now to Figures 24 and 26-28, the surgical attachment 300 may also include a shoulder 322 extending from and disposed around the driving member 312. The shoulder 322 may comprise or define various features described below. It is contemplated that in implementations where the driving member 312 does not include a shoulder 322, the driving member 312 may still include such features. The shoulder 322 of the driving member 312 may define a first cavity 324 that is in communication with the first opening 320 of the driving member 312. As shown in Figures 24 and 30, the first cavity 324 may resemble a canal defined in the shoulder 322 of the driving member 312, which merges with the first opening 320 defined in the driving member 312. As described further below, the first opening 320 and the first cavity 324 cooperate to support a lock assembly 326 for securing the surgical accessory tool 304 to the surgical attachment 300.

The lock assembly 326 includes a retention member 328 partially disposed in the first opening 320 of the driving member 312 for movement between a plurality of retention member 328 positions. Typically, the retention member 328 is cylindrically shaped such that the retention member 328 may be referred to as a cylindrical retention member 328. It is contemplated that the retention member 328 may comprise an alternative shape. Typically, at least a portion of the retention member 328 comprises a rounded surface. However, it is also contemplated that the retention member 328 may be without a rounded surface. In some implementations, a cross-section of the retention member 328 may comprise a polygonal shape. The plurality of retention member 328 positions includes a disengaged position (DP) where a first volume (V1) of the retention member 328 is disposed in the receiving bore 318 of the driving member 312. The disengaged position (DP) may be referred to as a first position. The plurality of retention member 328 positions also include an engaged position (EP) where a second volume (V2) of the retention member 328 is disposed in the receiving bore 318 of the driving member 312, with the second volume (V2) being less than the first volume (V1). The engaged position (EP) may be referred to as a second position. Said differently, when the retention member 328 is in the disengaged position (DP), a relatively larger amount of the retention member 328 is disposed in the receiving bore 318 and when the retention member 328 is in the engaged position (EP), a relatively lesser amount of the retention member 328 is disposed in the receiving bore 318. As described further below, the retention member 328 is in the disengaged position (DP) when the lock assembly 326 is not engaged with the surgical accessory tool 304. Conversely, the retention member 328 is in the engaged position (EP) when the lock assembly 326 is engaged within a groove 331 of the surgical accessory tool 304 to axially secure the surgical accessory tool 304 to the surgical attachment 300.

The lock assembly 326 may further include a first retention biasing member 332 at least partially disposed in the first cavity 324 of the shoulder 322 of the driving member 312. Although not required, as shown in Figures 24, 26, and 30, the first cavity 324 of the shoulder 322 containing the first biasing member 332 generally extends substantially parallel to the axis (A) of the driving member 312. Within the context of this disclosure, substantially parallel means +/- 5° from parallel. The first biasing member 332 may be any type of biasing member, provided that the selected biasing member is configured to urge the retention member 328 towards the disengaged position (DP). The retention member 328 in the disengaged position (DP) is disposed distally relative to the retention member 328 in the engaged position (DP). For example, the biasing member may be a spring or a wave spring. In the disengaged position (DP), the force exerted from the biasing member is the only force acting upon the retention member 328, which urges a relatively greater amount of the retention member 328 into the receiving bore 318 of the driving member 312. In the engaged position (EP), a surface defining the groove 331 of the surgical accessory tool 304 also exerts a force on the retention member 328, which urges the retention member 328 further into the first opening 320 and consequently reduces the volume of the retention member 328 within the receiving bore 318.

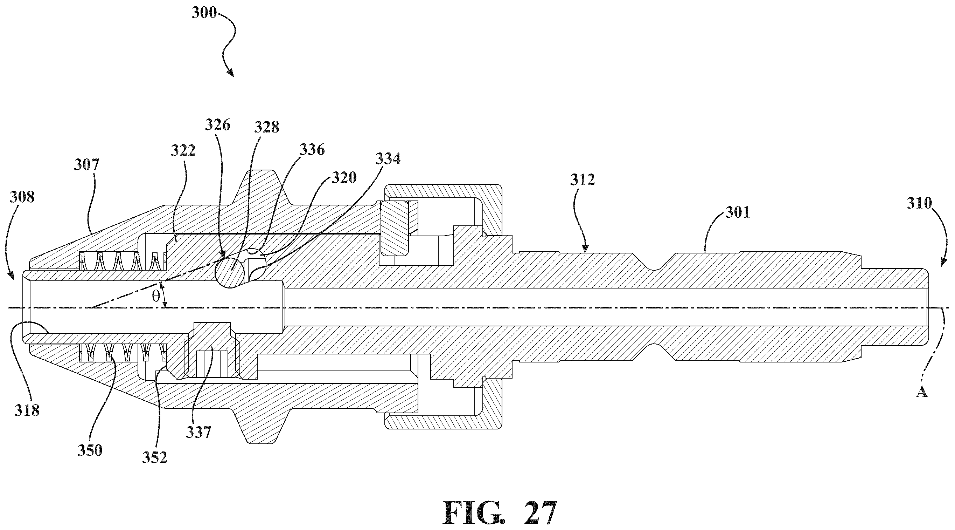

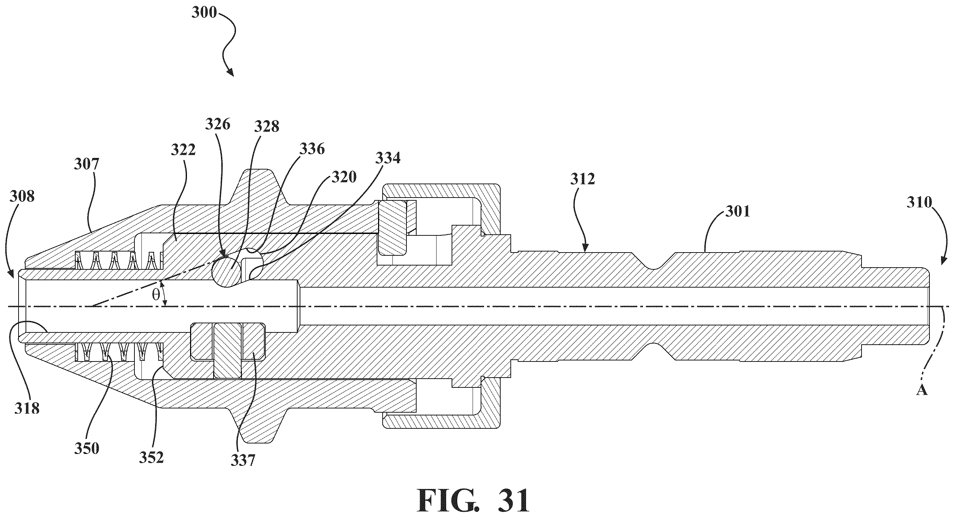

As shown in Figures 27 and 31, the driving member 312 may include a bottom surface 334 at least partially defining the first opening 320. The driving member 312 may also include a top planar surface 336, alternatively referred to as a ceiling 336, to further define the first opening 320. The top planar surface 336 that defines the first opening 320 may be angled relatively upward from a perspective beginning from the second end portion 310 (distal end portion) of the surgical attachment 300 extending towards the first end portion 308 (proximal end portion). The top planar surface may face the axis (A) and be disposed at an angle (Θ) between one and thirty degrees (between 1° and 30°) relative to the axis (A). In some implementations, the angle (Θ) is between five and fifteen degrees (between 5° and 15°). A distance between the bottom surface 334 of the first opening 320 and the axis (A) extending along the driving member 312 may decrease as the driving member 312 extends from the first end portion 308 toward the second end portion 310 adjacent the first opening 320. Alternatively, the relative size of the first opening 320 may decrease as the driving member 312 extends from the first end portion 308 (proximal end portion) towards the second end portion 310 (distal end portion) adjacent the opening 320. In this alternative configuration, the distance between the bottom of the first opening 320 and the ceiling 336 of the first opening 320 decreases as the driving member 312 extends from the first end portion 308 (proximal end portion) towards the second end portion 310 (distal end portion) adjacent the opening 320.

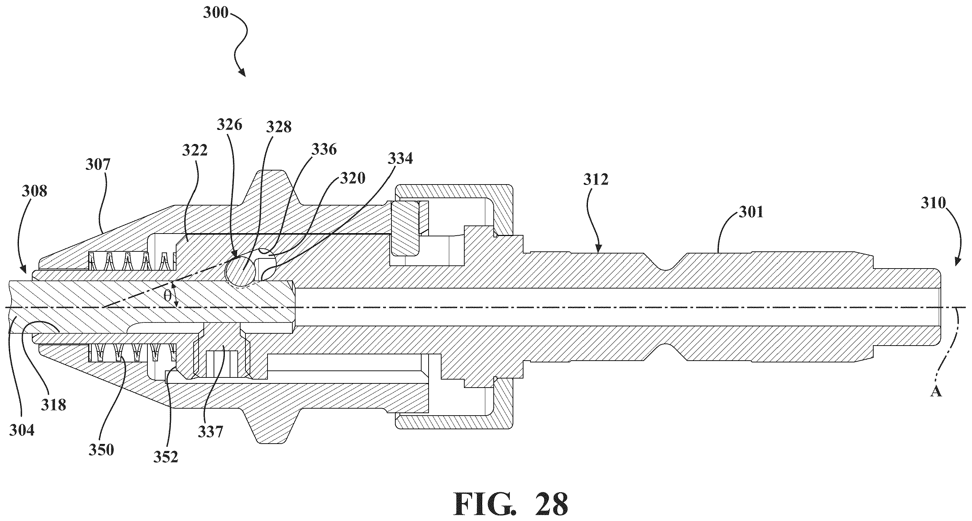

As shown in Figure 28, the retention member 328 in the engaged position (EP) may be spaced from the bottom surface 334 of the first opening 320 and in abutting contact with the groove 331 of the surgical accessory tool. Collectively, the force transferred from the biasing member 332 to the retention member 328, the force exerted from the flat region 330 of the surgical accessory tool 304, and a contact force from the top planar surface 336 of the first opening 320 are configured to cooperate and axially secure the surgical accessory tool 304 within the receiving bore 318 of the driving member 312.

Referring to Figures 24, 27, 30, and 31, exemplary drive elements 337 are shown to facilitate torque transmission between the surgical attachment 300 and the surgical accessory tool 304. In some implementations where there is a relatively large amount of torque being transferred between the surgical attachment 300 and the surgical attachment tool 304, the retention member 328 alone may not be sufficient to facilitate torque transfer. In such implementations, the surgical attachment 300 may include a drive element 337 coupled to the driving member 312 and disposed at least partially within the bore 318 of the driving member 312 opposite the axis (A) from the opening 320 of the driving member 312 to engage the flat region 330 of the surgical accessory tool 304 to transmit torque to the surgical accessory tool 304. The drive element 337 includes a flat that engages the flat region 330 of the surgical accessory tool 304 to facilitate torque transfer.

In one implementation shown in Figures 24 and 27, the drive element 337 comprises a set screw that may be set to a particular user defined depth within the bore 318 to permit the surgical accessory tool 304 to be received in the bore 318 to engage the retention member 328 with the groove 331 while also facilitating flat-to-flat engagement with the flat region 330 of the surgical accessory tool 304. In another implementation shown in Figures 30 and 31, the drive element 337 comprises a pin that may be set to a depth defined within the bore 318 to permit the surgical accessory tool 304 to be received in the bore 318 to engage the retention member 328 with the groove 331 while also facilitating flat-to-flat engagement with the flat region 330 of the surgical accessory tool 304. The pin may comprise a rectangular cross-section. In such an implementation, the driving member 312 may define a channel 339 for receiving drive element 337 and the drive element 337 may be press fit in the channel 339 or held in place within the channel 339 by a dowel. Other shapes are contemplated. As described further below, this cooperative configuration allows medical professionals to couple the surgical accessory tool 304 to the surgical attachment 300 with a single hand by merely aligning the flat region 330 of the surgical accessory tool 304 with the drive element 337 of the surgical attachment 300 (e.g., flat to flat) and inserting the surgical accessory tool 304 into the receiving bore 318 of the driving member 312 until the retention member 328 seats in the groove 331 of the surgical accessory tool 304.

Although not required or shown, the driving member 312 of the surgical attachment 300 may define a second opening, which may be symmetrically oriented relative to the first opening 320 such that the retention member 328 is disposed in both the first and second openings 320. In other words, the first and second openings 320 may be linearly aligned on opposite sides of the driving member 312 such that the first and second openings 320 are configured to receive the retention member 328. When the driving member 312 defines the second opening, the driving member 312 may also define a second cavity to support a second retention biasing member. The second cavity may be symmetrically oriented relative to the first cavity 324 across a reference plane that extends through the axis (A). For example, the reference plane may be the plane across which the section view of Figure 27 is taken. This configuration may advantageously be utilized to apply equal biasing forces on opposite sides of the retention member 328. This configuration may also include a first engagement pin 344 disposed partially within the first cavity 324 and partially within the first opening 320 between the first biasing member 332 and the retention member 328. The first engagement pin 344 may alternatively be referred to as a first ram 344. The first engagement pin 344 may be configured to transfer force from the first biasing member 332 to the retention member 328. The first engagement pin 344 may have various geometric configurations but is often cylindrically shaped, especially when the retention member 328 is cylindrical. Of course, a second engagement pin 346 may be similarly deployed between the second biasing member 342 and the retention member 328. Use of the first and/or second engagement pins 344, 346 advantageously ensures consistent force is applied from the first and/or second biasing members 332, 342 to the retention member 328.

As shown in Figures 22-24, the surgical attachment 300 may include a release collar 307 at the second end portion 310 of the attachment 300. As the name implies, the release collar 307 is configured to be actuated by a medical professional to release the surgical accessory tool 304. The release collar 307 is biased distally via a release biasing member 350 sandwiched between the release collar 307 and an outer surface 352 of the shoulder 322 of the driving member 312. The outer surface 352 of the shoulder 322 may be adjacent and spaced distally from the retention member 328. Similar to the first and/or second biasing members 332, 342, the release biasing member 350 may be any suitable biasing member, such as a spring or wave spring. To release the surgical accessory tool 304, the medical professional applies a force (i.e., an outside force) to the release collar 307 towards the first end portion 308 to overcome the biasing forces of the first and/or second retention biasing members 332, 342 to guide a contact surface 343 of the release collar 307 to abutting contact with the retention member 328. Continued force applied proximally to overcome the collective biasing forces of the first and/or second retention biasing members 332, 342 and the release biasing member 350 will urge the retention member 328 proximally. The contact surface 343 of the release collar 307 is configured to avoid contact with the shoulder 322 of the driving member 312. The release biasing member 350 is disposed distal the retention member 328.

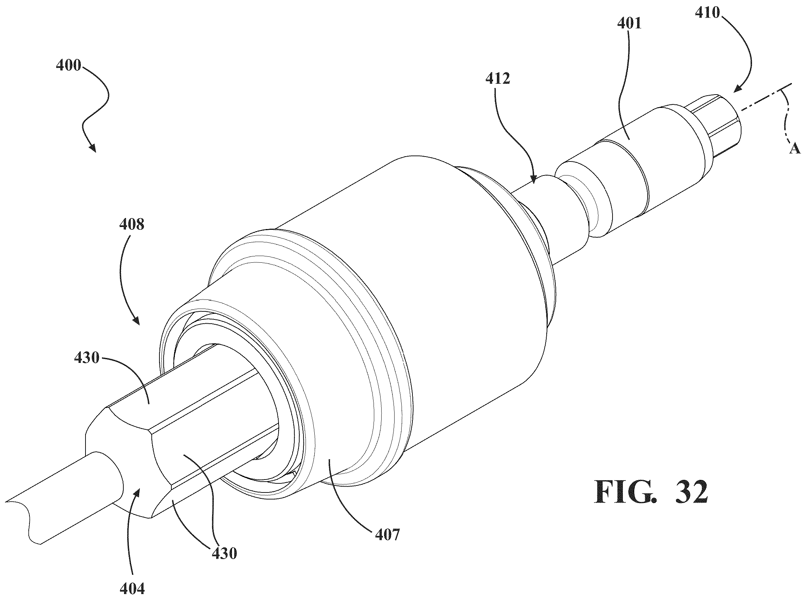

As shown throughout Figures 32-35, another implementation of surgical system 399 is provided. The surgical system 399 includes a surgical attachment 400 for coupling to a surgical driver 402 and a surgical accessory tool 404. In other words, the surgical attachment 400 couples the surgical driver 402 with the surgical accessory tool 404. The surgical driver 402 is not particularly limited. In some implementations, the surgical attachment 400 may also be referred to as a surgical assembly 400. In some implementations, the surgical attachment 400 and the surgical driver 402 may collectively form the surgical assembly 400, such that the surgical attachment 400 and the surgical driver 402 are integral. Similar to the rotary surgical handpiece 24 described above, suitable surgical drivers 402 include a body 26 and a motor 28 disposed within the body 26. The motor 28 includes an output shaft 30 configured to be rotated about an output axis (A) 32 in response to actuation of the motor 28. Beyond the structure described above, the configuration of the surgical driver 402 is not particularly limited for the purposes of this disclosure. For example, the surgical driver 402 may be the rotary surgical handpiece 24 describe above or the surgical drivers 402 described in U.S. Pat. Pub. No. 2007/0021766 or U.S. Patent No. 10,537,339, which are again expressly incorporated by reference in their entirety herein. Another suitable example of a surgical driver 302 is described in PCT Pub. No. WO2024/035819, published on February 15, 2024, and herein incorporated by reference in its entirety.

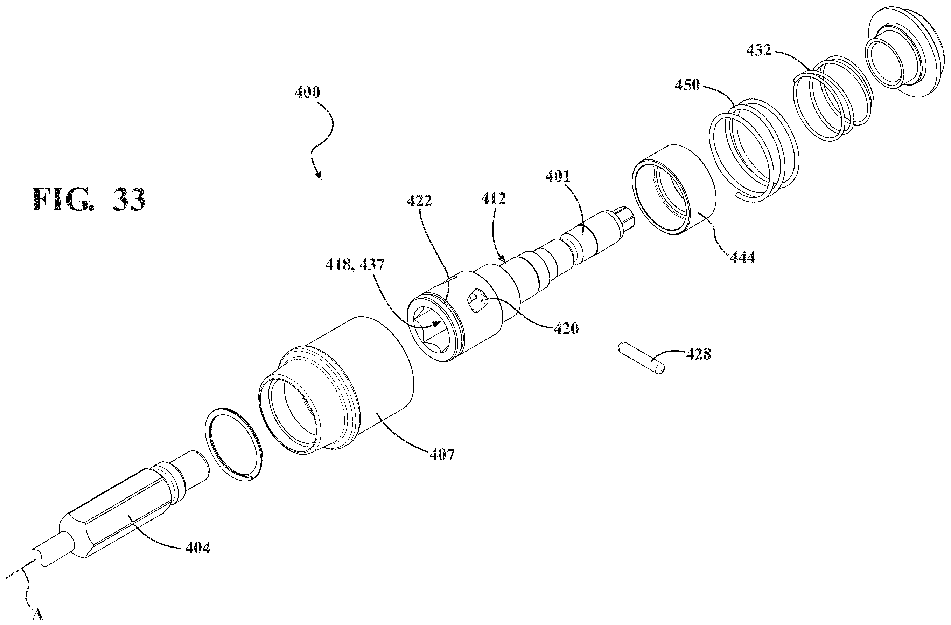

As shown in Figures 33-35, the surgical attachment 400 may include a driving member 412 being rotatable about an axis (A) and configured to transfer torque received from the surgical driver 402 to the surgical accessory tool 404. Similarly to the above-described surgical attachment 300, the surgical attachment 400 may not include a housing for coupling to the surgical driver 402. Instead, the driving member 412 may include an input shaft 401 that defines features for receiving torque from an output drive 403 of the surgical driver 402. Said differently, the surgical driver 402 may have a first coupler 403 and the driving member 412 may have a second coupler 401 adjacent a proximal end of the driving member 412. The second coupler 401 may be adapted for removable attachment to the first coupler 403 of the surgical driver 402. In this implementation of the surgical attachment 400, the entirety of the surgical attachment 400 may rotate in response to receiving torque from the surgical driver 402.

The input shaft 403 may also be referred to as a first end portion 408 of the surgical attachment 408 or a proximal end portion. The surgical attachment 400 may also include a second end portion 410 opposite the first end portion 408. The first end portion 408 may also be referred to as the proximal end portion and the second end portion 410 may be referred to as the distal end portion, with the distal end portion being relatively closer to the patient than the operator (typically a medical professional) of the surgical attachment 400 and the proximal end portion being relatively closer to the operator. In some implementations, the driving member 412 may be monolithic. In other words, the driving member may be of unitary construction. Such an implementation may reduce the number of components and provide for easier assembly of the surgical assembly 400.

Referring specifically to the driving member 412, those skilled the art may also refer to the driving member 412 as a drive shaft. The driving member 412 may define a receiving bore 418 extending along the axis (A) for receiving the surgical accessory tool 404. The driving member 412 also defines a first opening 420 extending transverse to and partially into the receiving bore 418. In other words, the driving member 412 includes a first opening 420 for providing access to the receiving bore 418.

Referring now to Figures 33-35, the surgical attachment 400 may also include a shoulder 422 extending from and disposed around the driving member 412. The shoulder 422 may comprise or define various features described below. It is contemplated that in implementations where the driving member 412 does not include a shoulder 422, the driving member 412 may still include such features.

The lock assembly 426 includes a retention member 428 partially disposed in the first opening 420 of the driving member 412 for movement between a plurality of retention member 428 positions. Typically, the retention member 428 is cylindrically shaped such that the retention member 428 may be referred to as a cylindrical retention member 428. It is contemplated that the retention member 428 may comprise an alternative shape. Typically, at least a portion of the retention member 428 comprises a rounded surface. The plurality of retention member 428 positions includes a disengaged position (DP) where a first volume (V1) of the retention member 428 is disposed in the receiving bore 418 of the driving member 412. The disengaged position (DP) may be referred to as a first position. The plurality of retention member 428 positions also include an engaged position (EP) where a second volume (V2) of the retention member 428 is disposed in the receiving bore 418 of the driving member 412, with the second volume (V2) being less than the first volume (V1). The engaged position (EP) may be referred to as a second position. Said differently, when the retention member 428 is in the disengaged position (DP), a relatively larger amount of the retention member 428 is disposed in the receiving bore 418 and when the retention member 428 is in the engaged position (EP), a relatively lesser amount of the retention member 428 is disposed in the receiving bore 418. As described further below, the retention member 428 is in the disengaged position (DP) when the lock assembly 426 is not engaged with the surgical accessory tool 404. Conversely, the retention member 428 is in the engaged position (EP) when the lock assembly 426 is engaged with a flat region 430 of the surgical accessory tool 404.

The lock assembly 426 may further include a first retention biasing member 432. The first retention biasing member 432 may be disposed around the outer surface of the driving member 412. The first biasing member 432 may be any type of biasing member, provided that the selected biasing member is configured to urge the retention member 428 towards the disengaged position (DP). The retention member 428 in the disengaged position (DP) is disposed distally relative to the retention member 428 in the engaged position (DP). For example, the biasing member may be a spring or a wave spring. In the disengaged position (DP), the force exerted from the biasing member is the only force acting upon the retention member 428, which urges a relatively greater amount of the retention member 428 into the receiving bore 418 of the driving member 412. In the engaged position (EP), the flat region 430 of the surgical accessory tool 404 also exerts a force on the retention member 428, which urges the retention member 428 further into the first opening 420 and consequently reduces the volume of the retention member 428 within the receiving bore 418.

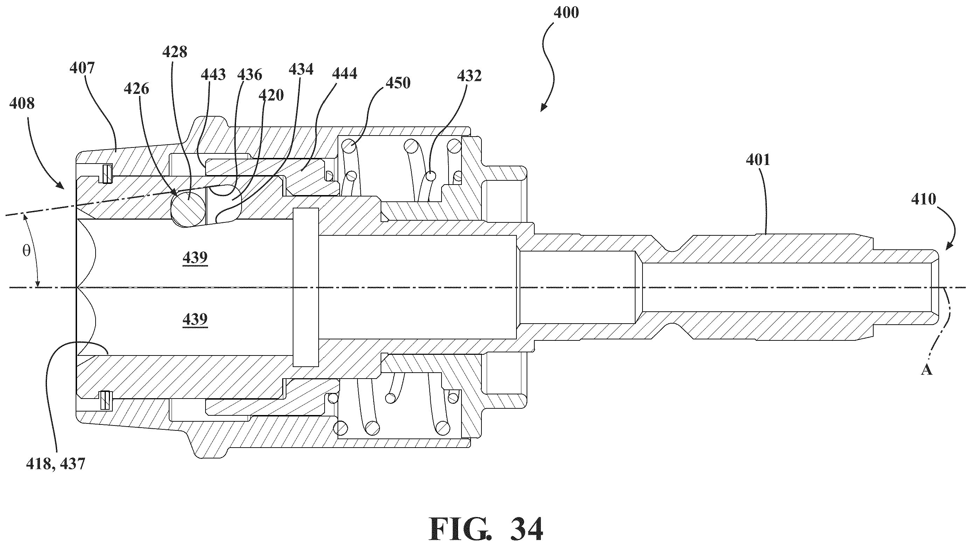

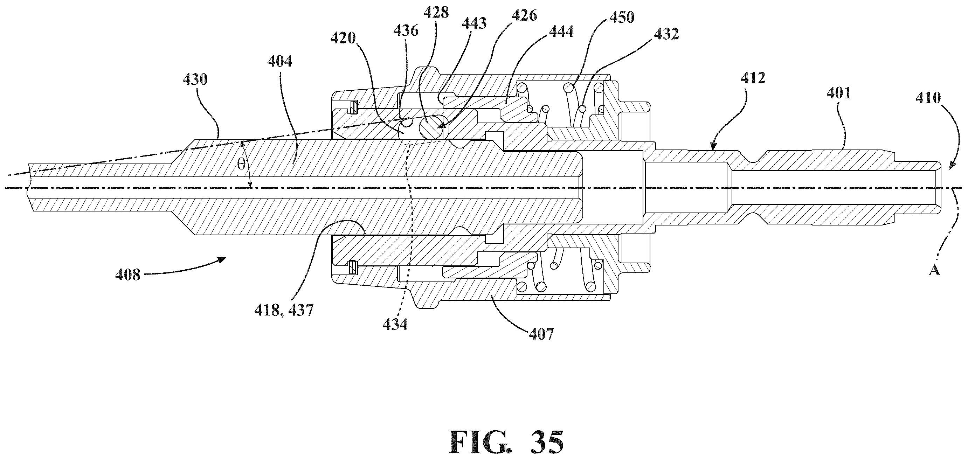

As shown in Figures 34 and 35, the driving member 412 may include a bottom surface 434 at least partially defining the first opening 420. The driving member 412 may also include a top planar surface 436, alternatively referred to as a ceiling 436, to further define the first opening 420. The top planar surface 436 that defines the first opening 420 may be angled relatively upward from a perspective beginning from the second end portion 410 (distal end portion) of the surgical attachment 400 extending towards the first end portion 408 (proximal end portion). Said differently, the top planar surface may face the axis (A) and be disposed at an angle (Θ) between one and thirty degrees (between 1° and 30°) relative to the axis (A). In some implementations, the angle (Θ) is between five and fifteen degrees (between 5° and 15°). A distance between the bottom surface 434 of the first opening 420 and the axis (A) extending along the driving member 412 may decrease as the driving member 412 extends from the first end portion 408 toward the second end portion 410 adjacent the first opening 420. Alternatively, the relative size of the first opening 420 may decrease as the driving member 412 extends from the first end portion 408 (proximal end portion) towards the second end portion 410 (distal end portion) adjacent the opening 420. In this alternative configuration, the distance between the bottom of the first opening 420 and the ceiling 436 of the first opening 420 decreases as the driving member 412 extends from the first end portion 408 (proximal end portion) towards the second end portion 410 (distal end portion) adjacent the opening 420.

As shown in Figure 35, the retention member 428 in the engaged position (EP) may be spaced from the bottom surface 434 of the first opening 420 and in abutting contact with the top planar surface 436 and the flat region 430 of the surgical accessory tool. Collectively, the force transferred from the biasing member 432 to the retention member 428, the force exerted from the flat region 430 of the surgical accessory tool 404, and a contact force from the top planar surface 436 of the first opening 420 are configured to cooperate and axially secure the surgical accessory tool 404 within the receiving bore 418 of the driving member 412. As described further below, this cooperative configuration allows medical professionals to couple the surgical accessory tool 404 to the surgical attachment 400 with a single hand by merely aligning the flat region 430 of the surgical accessory tool 404 with the retention member 428 and inserting the surgical accessory tool 404 into the receiving bore 418 of the driving member 412.

Referring to Figures 33 and 34, the driving member 412 may include a drive portion 437 to facilitate torque transmission between the surgical attachment 400 and the surgical accessory tool 404. The drive portion 437 may further define the bore 418. The drive portion 437 may include one or more planar surfaces 439 with at least one of the one or more planar surfaces 439 adapted to abut a complementary flat region 430 of the surgical accessory tool 404 to facilitate torque transfer. In one implementation shown in Figures 33 and 34, the drive portion 437 may define a hexagonal shape with six planar surfaces 439. In implementations where the surgical accessory tool 104 includes multiple flat regions 431, the retention member 428 may be adapted to abut any one of the flat regions 431 to axially secure the surgical accessory tool 404 to the surgical attachment 404. It is contemplated that the drive portion 437 may include five or fewer planar surfaces 439 or seven or more planar surfaces 439. In other implementations, the drive portion 437 may define a non-circular shape that corresponds with a like non-circular shape of the surgical accessory tool 404 to facilitate torque transfer.

Although not required, the driving member 412 of the surgical attachment 400 may define a second opening, which is symmetrically oriented relative to the first opening 420 such that the retention member 428 is disposed in both the first and second openings 420. In other words, the first and second openings 420 are linearly aligned on opposite sides of the driving member 412 such that the first and second openings 420 are configured to receive the retention member 428. This configuration may also include an engagement collar 444 disposed at least partially around the driving member 412 between the retention biasing member 432 and the retention member 428. The engagement collar 444 may be configured to transfer force from the biasing member 432 to the retention member 428. The engagement collar 444 may have various geometric configurations but is often tubularly shaped to accommodate the driving member 412 within an bore of the engagement collar 444.

As shown in Figures 33-35, the surgical attachment 400 may include a release collar 407 at the second end portion 410 of the attachment 400. As the name implies, the release collar 407 is configured to be actuated by a medical professional to release the surgical accessory tool 404. The release collar 407 is biased distally via a release biasing member 450. Similar to the retention biasing member 432, the release biasing member 450 may be any suitable biasing member, such as a spring or wave spring. To release the surgical accessory tool 404, the medical professional applies a force (i.e., an outside force) to the release collar 407 towards the first end portion 408 to overcome the biasing forces of the retention biasing members 432 to guide a contact surface 443 of the release collar 407 to abutting contact with the retention member 428. Continued force applied proximally to overcome the collective biasing forces of the retention biasing member 432 and the release biasing member 450 will urge the retention member 428 proximally. The contact surface 443 of the release collar 407 is configured to avoid contact with the shoulder 422 of the driving member 412. The release biasing member 450 may be disposed proximal the retention member 428.

Several different implementations of surgical attachments 200, 300, 400 for coupling and transferring torque to accessory tools 204, 304, 404 have been described above. The accessory tools 204, 304, 404 may be used for spinal surgical procedures in the drilling, reaming, and decorticating of bone and other bone-related tissue. The accessory tool for spinal surgical procedures (e.g., a screwdriver to place pedicle screws) may be removably coupled to the surgical attachments 200, 300, 400.

Several instances have been discussed in the foregoing description. However, the aspects discussed herein are not intended to be exhaustive or limit the disclosure to any particular form. Various modifications to these aspects will be readily apparent to those skilled in the art, and the generic principles defined herein may be applied to other aspects without departing from the scope of the disclosure. The terminology that has been used is intended to be in the nature of words of description rather than of limitation. Many modifications and variations are possible in light of the above teachings and the disclosure may be practiced otherwise than as specifically described.

The present disclosure also comprises the following clauses, with specific features laid out in dependent clauses, that may specifically be implemented as described in greater detail with reference to the configurations and drawings above.

I. A surgical sagittal saw comprising: a rotary surgical handpiece including a body, and a motor disposed in the body and including output shaft configured to be rotated about an output axis in response to actuation of the motor; and a sagittal saw attachment including: a housing operatively attached to the rotary surgical handpiece; a blade mount configured to releasably receive a surgical sagittal saw blade, the blade mount operatively attached to the housing and supported for oscillating motion about a pivot axis transverse to the output axis, and defining a lateral axis transverse to the pivot axis; and a drive assembly connected between the output shaft and the blade mount for converting rotary motion of the output shaft into oscillating motion of the blade mount about the pivot axis, the drive assembly including: an off-center rotation member operatively attached to the output shaft such that the off-center rotation member rotates about the output axis in response to actuation of the motor, the off-center rotation member defining a coupling void radially spaced from the output axis and concentric with a gimbal member axis which intersects the output axis and the pivot axis; a gimbal member extending along the gimbal member axis between: a first end portion supported within the coupling void such that the gimbal member is movable relative to the coupling void in only one degree of freedom such that the first end portion revolves around the output axis in response to actuation of the motor; and a second end portion coupled to the blade mount to oscillate the blade mount about the pivot axis, the second end portion configured to pivot relative to the lateral axis as the gimbal member oscillates the blade mount about the pivot axis in response to the first end portion of the gimbal member revolving around the output axis in response to actuation of the motor. II. The surgical sagittal saw according to clause I, further comprising a seal member arranged to contact an outer surface the off-center rotation member and an internal surface the housing. III. The surgical sagittal saw according to either of clauses I or II, wherein the second end portion of the gimbal member is coupled to the blade mount via at least one pin disposed along the lateral axis such that the second end portion of the gimbal member oscillates the blade mount about the pivot axis in response to the first end portion of the gimbal member revolving around the output axis in response to actuation of the motor. IV. The surgical sagittal saw according to clause III, further comprising at least one pin bearing member disposed between the at least one pin and the blade mount to permit rotation of the second end portion of the gimbal member relative to the blade mount about the lateral axis. V. The surgical sagittal saw according to any one of clauses I to IV, wherein the second end portion of the gimbal member defines a rim portion that at least partially surrounds the blade mount. VI. The surgical sagittal saw according to clause V, wherein a first lateral side of the rim portion defines a first pin void extending along the lateral axis, and a second lateral side of the rim portion, opposite the first lateral side, defines a second pin void extending along the lateral axis, and wherein the drive assembly further comprises a first pin member disposed in the first pin void and a second pin member disposed in the second pin void, each of the first pin member and the second pin member operatively attached to the blade mount such that the second end portion of the gimbal member oscillates the blade mount about the pivot axis in in response to the first end portion of the gimbal member revolving around the output axis in response to actuation of the motor. VII. The surgical sagittal saw according to clause VI, further comprising a first pin bearing member disposed between the first pin member and the first pin void and a second pin bearing member disposed between the second pin member and the second pin void, each of the first pin bearing member and the second pin bearing member permitting rotation of the second end portion of the gimbal member relative to the blade mount about the lateral axis. VIII. The surgical sagittal saw according to any one of clauses I to VII, wherein the gimbal member axis is offset from the output axis by at least 2 degrees. IX. The surgical sagittal saw according to any one of clauses I to VIII, wherein the blade mount is configured to pivot about the pivot axis along an arc of at least 4 degrees. X. The surgical sagittal saw according to any one of clauses I to IX, wherein the drive assembly further comprises one or more cylindrical bearing members disposed in the coupling void for supporting the first end portion of the gimbal member relative to the coupling void such that the gimbal member is movable relative to the coupling void in only one degree of freedom. XI. The surgical sagittal saw according to clause X, wherein the one or more cylindrical bearing members comprises a first cylindrical bearing member and a second cylindrical bearing member spaced from the first cylindrical bearing member. XII. The surgical sagittal saw according to one of clauses X or XI, wherein the one or more cylindrical bearing members comprise ball bearings.

XIII. A sagittal saw attachment for a rotary surgical handpiece having an output shaft configured to be rotated about an output axis in response to actuation of a motor, the sagittal saw attachment comprising: a housing configured to be operatively attached to the rotary surgical handpiece; a blade mount configured to releasably receive a surgical sagittal saw blade, the blade mount operatively attached to the housing and supported for oscillating motion about a pivot axis transverse to the output axis, and defining a lateral axis transverse to the pivot axis; and a drive assembly for converting rotary motion of the output shaft into oscillating motion of the blade mount about the pivot axis, the drive assembly including: an off-center rotation member configured to be operatively attached to the output shaft such that the off-center rotation member rotates about the output axis in response to actuation of the motor, the off-center rotation member defining a coupling void radially spaced from the output axis and concentric with a gimbal member axis which intersects the output axis and the pivot axis; a gimbal member extending along the gimbal member axis between: a first end portion supported within the coupling void such that the gimbal member is movable relative to the coupling void in only one degree of freedom such that the first end portion revolves around the output axis in response to actuation of the motor; and a second end portion coupled to the blade mount to oscillate the blade mount about the pivot axis, the second end portion configured to pivot relative to the lateral axis as the gimbal member oscillates the blade mount about the pivot axis in response to the first end portion of the gimbal member revolving around the output axis in response to actuation of the motor. XIV. The sagittal saw attachment according to clause XIII, further comprising a seal member arranged to contact an outer surface the off-center rotation member and an internal surface the housing. XV. The sagittal saw attachment according to one of clauses XIII or XIV, wherein the second end portion of the gimbal member is coupled to the blade mount via at least one pin disposed along the lateral axis such that the second end portion of the gimbal member oscillates the blade mount about the pivot axis in response to the first end portion of the gimbal member revolving around the output axis in response to actuation of the motor. XVI. The sagittal saw attachment according to clause XV, further comprising at least one pin bearing member disposed between the at least one pin and the blade mount to permit rotation of the second end portion of the gimbal member relative to the blade mount about the lateral axis. XVII. The sagittal saw attachment according to any one of clauses XIII to XVI, wherein the second end portion of the gimbal member defines a rim portion that at least partially surrounds the blade mount. XVIII. The sagittal saw attachment according to clause XVII, wherein a first lateral side of the rim portion defines a first pin void extending along the lateral axis, and a second lateral side of the rim portion, opposite the first lateral side, defines a second pin void extending along the lateral axis, and wherein the drive assembly further comprises a first pin member disposed in the first pin void and a second pin member disposed in the second pin void, each of the first pin member and the second pin member operatively attached to the blade mount such that the second end portion of the gimbal member oscillates the blade mount about the pivot axis in in response to the first end portion of the gimbal member revolving around the output axis in response to actuation of the motor. XIX. The sagittal saw attachment according to clause XVIII, further comprising a first pin bearing member disposed between the first pin member and the first pin void and a second pin bearing member disposed between the second pin member and the second pin void, each of the first pin bearing member and the second pin bearing member permitting rotation of the second end portion of the gimbal member relative to the blade mount about the lateral axis. XX. The sagittal saw attachment according to any one of clauses XIII to XIX, wherein the gimbal member axis is offset from the output axis by at least 2 degrees. XXI. The sagittal saw attachment according to any one of clauses XIII to XX, wherein the blade mount is configured to pivot about the pivot axis along an arc of at least 4 degrees. XXII. The sagittal saw attachment according to any one of clauses XIII to XXI, wherein the drive assembly further comprises one or more cylindrical bearing members disposed in the coupling void for supporting the first end portion of the gimbal member relative to the coupling void such that the gimbal member is movable relative to the coupling void in only one degree of freedom. XXIII. The sagittal saw attachment according to clause XXII, wherein the one or more cylindrical bearing members comprises a first cylindrical bearing member and a second cylindrical bearing member spaced from the first cylindrical bearing member. XIV. The sagittal saw attachment according to one of clauses XXII or XXIII, wherein the one or more cylindrical bearing members comprise ball bearings.

XXV. A drive assembly configured to be connected between an output shaft of a motor defining an output axis and a tool mount for converting rotary motion of the output shaft into oscillating motion of the tool mount about a pivot axis, the drive assembly comprising: an off-center rotation member configured to be operatively attached to the output shaft such that the off-center rotation member rotates about the output axis in response to actuation of the motor, the off-center rotation member defining a coupling void radially spaced from the output axis and concentric with a gimbal member axis which intersects the output axis and the pivot axis; one or more cylindrical bearing members disposed in the coupling void; a gimbal member extending along the gimbal member axis between: a first end portion supported within the coupling void by the one or more cylindrical bearing members such that the first end portion rotates relative to the coupling void about the gimbal member axis and revolves around the output axis in response to actuation of the motor; and a second end portion coupled to the tool mount to oscillate the tool mount about the pivot axis, the second end portion configured to pivot relative to a lateral axis as the gimbal member oscillates the tool mount about the pivot axis in response to the first end portion of the gimbal member revolving around the output axis in response to actuation of the motor. XXVI. The drive assembly according to clause XXV, wherein the second end portion of the gimbal member is coupled to the tool mount via at least one pin disposed along the lateral axis such that the second end portion of the gimbal member oscillates the tool mount about the pivot axis in response to the first end portion of the gimbal member revolving around the output axis in response to actuation of the motor. XXVII. The drive assembly according to clause XXVI, further comprising at least one pin bearing member disposed between the at least one pin and the tool mount to permit rotation of the second end portion of the gimbal member relative to the tool mount about the lateral axis. XXVIII. The drive assembly according to any one of clauses XXV to XXVII, wherein the second end portion of the gimbal member defines a rim portion that at least partially surrounds the tool mount. XXIX. The drive assembly according to clause XXVIII, wherein a first lateral side of the rim portion defines a first pin void extending along the lateral axis, and a second lateral side of the rim portion, opposite the first lateral side, defines a second pin void extending along the lateral axis, and wherein the drive assembly further comprises a first pin member disposed in the first pin void and a second pin member disposed in the second pin void, each of the first pin member and the second pin member operatively attached to the tool mount such that the second end portion of the gimbal member oscillates the tool mount about the pivot axis in in response to the first end portion of the gimbal member revolving around the output axis in response to actuation of the motor. XXX. The drive assembly according to clause XXIX, further comprising a first pin bearing member disposed between the first pin member and the first pin void and a second pin bearing member disposed between the second pin member and the second pin void, each of the first pin bearing member and the second pin bearing member permitting rotation of the second end portion of the gimbal member relative to the tool mount about the lateral axis. XXXI. The drive assembly according to any one of clauses XXV to XXX, wherein the one or more cylindrical bearing members comprises a first cylindrical bearing member and a second cylindrical bearing member spaced from the first cylindrical bearing member. XXXII. The drive assembly according to any one of clauses XXV to XXXI, wherein the one or more cylindrical bearing members comprise ball bearings. XXXIII. The drive assembly according to any one of clauses XXV to XXXII, wherein the gimbal member axis is offset from the output axis by at least 2 degrees. XXXIV. The drive assembly according to any one of clauses XXV to XXXIII, wherein the tool mount is configured to pivot about the pivot axis along an arc of at least 4 degrees.

XXXV. A sagittal saw system comprising: a saw blade extending between a distal portion and a proximal portion defining an engagement slot; a blade mount for releasably receiving the saw blade, the blade mount defining a blade channel sized to at least partially receive the saw blade; and a cylindrical retention member supported by the blade mount for movement between a plurality of retention member positions including: an engaged position where the cylindrical retention member abuts the engagement slot of the saw blade to retain the saw blade within the blade channel, and a disengaged position where the cylindrical retention member is spaced from the engagement slot of the saw blade to permit a user to translate the saw blade distally to remove the saw blade from the blade channel. XXXVI. The sagittal saw system according to clause XXXV, wherein the saw blade includes a first surface and an opposing second surface, wherein the engagement slot extends between the first surface and the second surface, and wherein the engagement slot bounded by a distal wall, a proximal wall, and opposing lateral walls extending therebetween. XXXVII. The sagittal saw system according to clause XXXVI, wherein the proximal wall extends between a first edge and a second edge and is arranged at an obtuse angle relative to the second surface such that the first edge is closer to the distal portion than the second edge. XXXVIII. The sagittal saw system according to one of clauses XXXVI or XXXVII, wherein in the engaged position, the cylindrical retention member abuts the proximal wall of the saw blade to retain the saw blade within the blade channel. XXXIX. The sagittal saw system according to any one of clauses XXXV to XXXVIII, wherein the blade mount comprises a stem member extending between a first end portion and a second end portion, wherein the second end portion defines a first stem surface and a ramp portion extending between a first ramp edge and a second ramp edge, the ramp portion arranged at an acute angle relative to the first stem surface such that the first ramp edge is arranged distal to the second ramp edge, and wherein the cylindrical retention member arranged for movement along the ramp portion between the plurality of retention member positions. XL. The sagittal saw system according to clause XXXIX, wherein the blade mount further comprises a cap coupled to the second end portion of the stem member such that the cap and the first stem surface of the stem member cumulatively define the blade channel. XLI. The sagittal saw system according to one of clauses XXXIX or XL, further comprising a motor operatively attached to the first end portion of the stem member to oscillate the blade mount and the saw blade. XLII. The sagittal saw system according to any one of clauses XXXV to XLI, further comprising a biasing member supported by the blade mount and operatively attached to the cylindrical retention member to urge the cylindrical retention member to the engaged position. XLIII. The sagittal saw system according to clause XLII, wherein the proximal portion of the saw blade includes a proximal blade wall, and wherein the proximal blade wall is configured to abut the cylindrical retention member in response to insertion of the saw blade into the blade channel such that the proximal blade wall moves the cylindrical retention member toward the disengaged position. XLIV. The sagittal saw system according to clause XLIII, wherein the biasing member is configured to urge the cylindrical retention member to the engaged position in response to the saw blade being fully inserted into the blade channel to retain the saw blade within the blade channel. XLV. The sagittal saw system according to any one of clauses XXXV to XLIV, further comprising a release member operatively attached to the blade mount and arranged for movement relative to the blade mount such that the release member is configured to abut the cylindrical retention member to move the cylindrical retention member from the engaged position to the disengaged position in response to user engagement. XLVI. The sagittal saw system according any one of clauses XXXV to XLV, wherein the proximal portion of the saw blade includes a proximal blade wall and two lateral blade walls, and wherein the proximal portion of the saw blade defines a tapered blade wall between the proximal blade wall and each of the lateral blade walls. XLVII. The sagittal saw system according to clause XLVI, wherein the blade channel defines tapered channel walls corresponding to the tapered blade walls and configured to abut the tapered blade walls to align the saw blade relative to the blade mount.

XLVIII. A sagittal saw system comprising: a saw blade having a first surface and an opposing second surface and extending between a distal portion and a proximal portion defining an engagement slot extending between the first surface and the second surface, the engagement slot bounded by a distal wall, a proximal wall, and opposing lateral walls extending therebetween, wherein the proximal wall extends between a first edge and a second edge and is arranged at an obtuse angle relative to the second surface such that the first edge is closer to the distal portion than the second edge; and a blade mount for releasably receiving the saw blade, the blade mount including: a stem member extending between a first end portion and a second end portion, wherein the second end portion defines a first stem surface and a ramp portion extending between a first ramp edge and a second ramp edge, the ramp portion arranged at an acute angle relative to the first stem surface such that the first ramp edge is arranged distal to the second ramp edge; a cap coupled to the second end portion of the stem member such that the cap and the first stem surface of the stem member cumulatively define a blade channel sized to at least partially receive the saw blade; and a retention member arranged for movement along the ramp portion between a plurality of retention member positions including: an engaged position where the retention member abuts the proximal wall of the saw blade to retain the saw blade within the blade channel, and a disengaged position where the retention member is spaced from the proximal wall of the saw blade to permit a user to translate the saw blade distally to remove the saw blade from the blade channel. XLIX. The sagittal saw system according to clause XLVIII, wherein the retention member is spherical. L. The sagittal saw system according to clause XLVIII, wherein the retention member is cylindrical. LI. The sagittal saw system according to any one of clauses XLVIII to L, further comprising a motor operatively attached to the first end portion of the stem member to oscillate the blade mount and the saw blade. LII. The sagittal saw system according to any one of clauses XLVIII to LII, further comprising a biasing member supported by the blade mount and operatively attached to the retention member to urge the retention member to the engaged position. LIII. The sagittal saw system according to clause LII, wherein the proximal portion of the saw blade includes a proximal blade wall, and wherein the proximal blade wall is configured to abut the retention member in response to insertion of the saw blade into the blade channel such that the proximal blade wall moves the retention member toward the disengaged position. LIV. The sagittal saw system according to clause LIII, wherein the biasing member is configured to urge the retention member to the engaged position in response to the saw blade being fully inserted into the blade channel to retain the saw blade within the blade channel. LV. The sagittal saw system according to any one of clauses XLVIII to LIV, further comprising a release member operatively attached to the blade mount and arranged for movement relative to the blade mount such that the release member is configured to abut the retention member to move the retention member from the engaged position to the disengaged position in response to user engagement. LVI. The sagittal saw system according any one of clauses XLVIII to LV, wherein the proximal portion of the saw blade includes a proximal blade wall and two lateral blade walls, and wherein the proximal portion of the saw blade defines a tapered blade wall between the proximal blade wall and each of the lateral blade walls. LVII. The sagittal saw system according to clause LVI, wherein the blade channel defines tapered channel walls corresponding to the tapered blade walls and configured to abut the tapered blade walls to align the saw blade relative to the blade mount.

LVIII. A surgical attachment for coupling to a surgical driver and a surgical accessory tool, the surgical attachment comprising: a housing having a first end portion for coupling to the surgical driver and a second end portion opposite the first end portion; a driving member at least partially disposed within the housing, the driving member being rotatable about an axis relative to the housing and configured to transfer torque received from the surgical driver to the surgical accessory tool, the driving member comprising, a drive portion defining (i) a receiving bore extending along the axis for receiving the surgical accessory tool and (ii) a first opening extending transverse to and partially into the receiving bore, and a shoulder extending from and disposed around the drive portion, the shoulder defining a first cavity in communication with the first opening of the drive portion; and a lock assembly for securing the surgical accessory tool, the lock assembly comprising, a cylindrical retention member partially disposed in the first opening of the drive portion for movement between a plurality of retention member positions including, a disengaged position where a first volume of the cylindrical retention member is disposed in the receiving bore of the drive portion, and an engaged position where a second volume of the cylindrical retention member is disposed in the receiving bore of the drive portion, with the second volume being less than the first volume, and a first biasing member disposed in the first cavity of the shoulder and configured to urge the cylindrical retention member towards the disengaged position. LIX. The surgical attachment of clause LVIII wherein the first opening of the drive portion, first cavity of the shoulder, and the first biasing member cooperate to secure the surgical accessory tool. LX. The surgical attachment of clause LVIII wherein a portion of the shoulder defining the first opening defines a bottom surface and further defines a ceiling opposite the bottom surface, and wherein a distance between the bottom surface and the axis of the driving member decreases as the driving member extends from a proximate end to a distal end. LXI. The surgical attachment of clause LX wherein the first cavity of the shoulder containing the first biasing member extends substantially parallel to the axis of the driving member. LXII. The surgical attachment of clause LX or LXI wherein the cylindrical retention member in the engaged position is spaced from the bottom surface of the first opening and in contact with the ceiling. LXIII. The surgical attachment of any one of clauses LVIII to LXII wherein the shoulder defines a second opening symmetrically oriented relative to the first opening such that the cylindrical retention member is disposed in the first and second openings. LXIV. The surgical attachment of clauses LXIII wherein the shoulder further defines a second cavity symmetrically oriented relative to the first cavity, and wherein the lock assembly comprises a second biasing member disposed in the second cavity of the shoulder, with the second biasing member also configured to urge the cylindrical retention member towards the disengaged position. LXV. The surgical attachment of any one of clauses LXIII to LXIV wherein the lock assembly further comprises a first ram disposed partially within the first cavity and partially within the first opening between the first biasing member and the cylindrical retention member, with the first ram configured to transfer force from the first biasing member to the cylindrical retention member. LXVI. The surgical attachment of any one of clauses LVIII to LXV wherein: the housing comprises a release collar at the second end portion; the shoulder defines an outer surface adjacent and spaced from the cylindrical retention member; and the surgical attachment further comprises a release biasing member sandwiched between the release collar and the outer surface of the shoulder, and the release collar defines a contact surface configured to avoid contact with the shoulder and contact the cylindrical biasing member to urge the cylindrical biasing member into the disengaged position when the release collar is acted on by an outside force.

LXVII. A surgical handpiece system comprising: a surgical driver including a motor for generating torque; an AO drill bit having a shaft including at least one flat region; and a surgical attachment for coupling to the surgical driver and AO drill bit, the surgical attachment comprising: a housing having a first end portion for coupling to the surgical driver and a second end portion opposite the first end portion; a driving member at least partially disposed within the housing, the driving member being rotatable about an axis relative to the housing and configured to transfer torque received from the surgical driver, the driving member comprising, a drive portion defining (i) a receiving bore extending along the axis and (ii) a first opening extending transverse to and partially into the receiving bore, and a shoulder extending from and disposed around the drive portion, the shoulder defining a first cavity in communication with the first opening of the drive portion; and a lock assembly for securing the AO drill bit, the lock assembly comprising, a retention member partially disposed in the first opening of the drive portion for movement between a plurality of retention member positions including, a disengaged position where a first volume of the retention member is disposed in the receiving bore of the drive portion, and an engaged position where a second volume of the retention member is disposed in the receiving bore of the drive portion, with the second volume being less than the first volume, and a first biasing member disposed in the first cavity of the shoulder and configured to urge the retention member towards the disengaged position when contacted by the retention member; and wherein the surgical handpiece system is configured to secure the AO drill bit in the receiving bore when the flat region of the shaft of the AO drill bit is aligned with the retention member of the lock assembly, and wherein the surgical handpiece system is configured to prevent the AO drill bit from being secured in the receiving bore when the flat region of the shaft is misaligned with the retention member. LXVIII. The surgical handpiece system of clause LXVII wherein the first opening of the drive portion, first cavity of the shoulder, and the first biasing member cooperate to secure the AO drill bit. LXIX. The surgical handpiece system of clause LXVIII wherein a portion of the shoulder defining the first opening defines a bottom surface and further defines a ceiling opposite the bottom surface, and wherein a distance between the bottom surface and the axis of the driving member decreases as the driving member extends from a proximate end to a distal end. LXX. The surgical handpiece system of clause LXIX wherein the first cavity of the shoulder containing the first biasing member extends substantially parallel to the axis of the driving member. LXXI. The surgical handpiece system of clause LXIX or LXX wherein the retention member is cylindrical. LXXII. The surgical handpiece system of any one of clauses LXVII to LXXI wherein the shoulder defines a second opening symmetrically oriented relative to the first opening such that the retention member is partially disposed in the first and second openings. LXXIII. The surgical handpiece system of clauses LXXII wherein the shoulder further defines a second cavity symmetrically oriented relative to the first cavity, and wherein the lock assembly comprises a second biasing member disposed in the second cavity of the shoulder, with the second biasing member also configured to urge the retention member towards the disengaged position. LXXIV. The surgical handpiece system of any one of clauses LXVII to LXXIII wherein the lock assembly further comprises a first ram disposed partially within the first cavity and partially within the first opening between the first biasing member and the retention member, with the first ram configured to transfer force from the first biasing member to the retention member. LXXV. The surgical handpiece system of any one of clauses LXVII to LXXIV wherein: the housing comprises a release collar at the second end portion; the shoulder defines an outer surface adjacent and spaced from the retention member; and the surgical attachment further comprises a release biasing member sandwiched between the release collar and the outer surface of the shoulder, and the release collar defines a contact surface configured to avoid contact with the shoulder and contact the cylindrical biasing member to urge the cylindrical biasing member into the disengaged position when the release collar is acted on by an outside force.
The present disclosure also provides for the following examples:
1. A surgical assembly for coupling to a surgical accessory tool, the surgical assembly comprising:
   a driving member extending along an axis from a proximal end to a distal end, the driving member being rotatable about an axis and configured to transfer torque to the surgical accessory tool, the driving member defining:
      (i) a receiving bore extending along the axis for receiving the surgical accessory tool,
      (ii) an opening extending transverse to and partially into the receiving bore, and
      (iii) a cavity in communication with the opening; and
   a lock assembly for securing the surgical accessory tool to the driving member, the lock assembly comprising,
   a retention member partially disposed in the opening of the driving member for movement between a plurality of retention member positions including,
   a first position where a first volume of the retention member is disposed in the receiving bore of the driving member, and
   a second position where a second volume of the retention member is disposed in the receiving bore of the driving member, with the second volume being less than the first volume, and
   a retention biasing member at least partially disposed in the cavity of the driving member and configured to urge the retention member towards the first position for engaging the surgical accessory tool.
2. The surgical assembly of example 1, further comprising a housing, wherein at least a portion of the driving member is disposed within the housing.
3. The surgical assembly of example 2, wherein the housing comprises a coupler configured to be removably attached to a surgical driver.
4. The surgical assembly of example 1, wherein the driving member comprises a coupler adjacent the proximal end configured to be removably attached to a surgical driver.
5. The surgical assembly of any one of examples 1-4, wherein the retention member is cylindrical.
6. The surgical assembly of any one of examples 1-5, wherein the driving member is monolithic.
7. The surgical assembly of any one of examples 1-6, further comprising a drive element coupled to the driving member and disposed at least partially within the bore of the driving member opposite the axis from the opening of the driving member and configured to engage a flat region of the surgical accessory tool to transmit torque to the surgical accessory tool.
8. The surgical assembly of examples 7, wherein the drive element comprises one element selected from a set screw and a pin.
9. The surgical assembly of any one of examples 1-8, wherein the retention member in the first position is disposed axially distal to the retention member in the second position.
10. The surgical assembly of any one of examples 1-9, wherein the driving member includes a planar surface at least partially defining the opening, the planar surface facing the axis and being disposed at an angle between one and thirty degrees relative to the axis.
11. The surgical assembly of examples 10, wherein the planar surface is disposed at an angle between five and fifteen degrees relative to the axis.
12. The surgical assembly of any one of examples 10 and 11, wherein the driving member further comprises a bottom surface at least partially defining the opening, the bottom surface being disposed opposite and facing the planar surface, and wherein a distance between the bottom surface and the axis of the driving member decreases as the driving member extends from a proximal end to a distal end.
13. The surgical assembly of example 12, wherein the retention member in the second position is spaced from the bottom surface of the opening and in contact with the planar surface.
14. The surgical assembly of any one of examples 1-13, wherein the cavity of the driving member extends parallel to the axis of the driving member.
15. The surgical assembly of any one of examples 1-14, wherein the lock assembly further comprises an engagement pin disposed at least partially within the cavity and at least partially within the opening between the retention biasing member and the retention member, with the engagement pin adapted to transfer force from the retention biasing member to the retention member.
16. The surgical assembly of any one of examples 1-15, further comprising a release collar slidably coupled to the driving member and a release biasing member disposed between the release collar and the driving member to bias the release collar away from the retention member, and wherein the release collar is slidable to abut the retention member to move the retention member to the second position in response to a force applied to the release collar sufficient to overcome opposing forces of the release biasing member and the retention biasing member.
17. The surgical assembly of example 16, wherein the release biasing member is disposed distal the retention member.
18. The surgical assembly of any one of examples 1-17, wherein the opening is further defined as a first opening and the driving member further defines a second opening symmetrically oriented relative to the first opening such that the retention member is disposed in the first and second openings.
19. The surgical assembly of any one of examples 1-18, wherein the cavity is further defined as a first cavity and wherein the retention biasing member is further defined as a first retention biasing member, and wherein the driving member further defines a second cavity symmetrically oriented relative to the first cavity about a reference plane that extends through the axis, and wherein the lock assembly comprises a second retention biasing member disposed in the second cavity of the driving member, with the second biasing member also configured to urge the retention member toward the first position.
20. A surgical assembly for coupling to a surgical accessory tool, the surgical assembly comprising:
   a driving member extending along an axis from a proximal end to a distal end, the driving member being rotatable about an axis and configured to transfer torque to the surgical accessory tool, the driving member defining (i) a receiving bore extending along the axis for receiving the surgical accessory tool, and (ii) an opening extending transverse to and partially into the receiving bore, with the driving member having a planar surface at least partially defining the opening, the planar surface facing the axis and being disposed at an angle between one and thirty degrees relative to the axis; and
   a lock assembly for securing the surgical accessory tool to the driving member, the lock assembly comprising,
   a retention member partially disposed in the opening of the driving member for movement between a plurality of retention member positions including,
   a first position where a first volume of the retention member is disposed in the receiving bore of the driving member, and
   a second position where a second volume of the retention member is disposed in the receiving bore of the driving member, with the second volume being less than the first volume, and
   a retention biasing member coupled to the driving member and configured to urge the retention member towards the first position for engaging the surgical accessory tool.
21. The surgical assembly of example 20, further comprising a housing, wherein at least a portion of the driving member is disposed within the housing.
22. The surgical assembly of example 21, wherein the housing comprises a coupler configured to be attached to a surgical driver.
23. The surgical assembly of example 20, wherein the driving member comprises a coupler adjacent the proximal end configured to be removably attached to a surgical driver.
24. The surgical assembly of any one of examples 20-23, wherein the retention member is cylindrical.
25. The surgical assembly of any one of examples 20-24, wherein the driving member is monolithic.
26. The surgical assembly of any one of examples 20-25, further comprising a drive element coupled to the driving member and disposed at least partially within the bore of the driving member opposite the axis from the opening of the driving member and configured to engage a flat region of the surgical accessory tool to transmit torque to the surgical accessory tool.
27. The surgical assembly of example 26, wherein the drive element comprises one element selected from a set screw and a pin.
28. The surgical assembly of any one of examples 20-27, wherein the retention member in the first position is disposed axially distal to the retention member in the second position.
29. The surgical assembly of any one of examples 20-28, wherein the planar surface is disposed at an angle between five and fifteen degrees relative to the axis.
30. The surgical assembly of any one of examples 20-29, wherein the driving member further comprises a bottom surface at least partially defining the opening, the bottom surface being disposed opposite and facing the planar surface, and wherein a distance between the bottom surface and the axis of the driving member decreases as the driving member extends from a proximal end to a distal end.
31. The surgical assembly of example 30, wherein the retention member in the second position is spaced from the bottom surface of the opening and in contact with the planar surface.
32. The surgical assembly of any one of examples 20-31, wherein the lock assembly further comprises an engagement pin disposed at least partially within a cavity of the driving member and at least partially within the opening between the retention biasing member and the retention member, with the engagement pin adapted to transfer force from the retention biasing member to the retention member.
33. The surgical assembly of any one of examples 20-31, wherein the lock assembly further comprises an engagement collar disposed at least partially around the driving member between the retention biasing member and the retention member, with the engagement collar adapted to transfer force from the retention biasing member to the retention member.
34. The surgical assembly of any one of examples 20-33, further comprising a release collar slidably coupled to the driving member and a release biasing member disposed between the release collar and the driving member to bias the release collar away from the retention member, and wherein the release collar is slidable to abut the retention member to move the retention member to the second position in response to a force applied to the release collar sufficient to overcome opposing forces of the release biasing member and the retention biasing member.
35. The surgical assembly of example 34, wherein the release biasing member is disposed distal the retention member.
36. The surgical assembly of example 34, wherein the release biasing member is disposed proximal the retention member.
37. The surgical assembly of any one of examples 20-36, wherein the opening is further defined as a first opening and the driving member further defines a second opening symmetrically oriented relative to the first opening such that the retention member is disposed in the first and second openings.
38. A surgical system comprising:
   a surgical accessory tool including at least one flat region and a groove opposite the flat region; and
   a surgical assembly adapted for removably coupling with the surgical accessory tool, the surgical assembly comprising,
   a driving member extending along an axis from a proximal end to a distal end, the driving member being rotatable about an axis and configured to transfer torque to the surgical accessory tool, the driving member defining:
      (i) a receiving bore extending along the axis for receiving the surgical accessory tool, and
      (ii) an opening extending transverse to and partially into the receiving bore,
   a drive element coupled to the driving member and disposed at least partially within the bore of the driving member opposite the axis from the opening of the driving member to engage the flat region of the surgical accessory tool to transmit torque to the surgical accessory tool,
   a lock assembly coupled to the driving member to axially secure the surgical accessory tool to the driving member, the lock assembly comprising,
   a retention member partially disposed in the opening of the driving member for movement between a plurality of retention member positions including,
   a first position where a first volume of the retention member is disposed in the receiving bore of the driving member, and
   a second position axially proximal relative to the first position, where a second volume of the retention member is disposed in the receiving bore of the driving member, with the second volume being less than the first volume, and with a portion of the retention member being disposed in the groove of the surgical accessory tool to axially secure the surgical accessory tool to the driving member, and
   a retention biasing member coupled to the driving member and configured to urge the retention member towards the first position.
39. The surgical system of example 38, further comprising a surgical driver having a first coupler, and wherein the driving member comprises a second coupler adjacent the proximal end of the driving member adapted for removable attachment to the first coupler of the surgical driver.
40. The surgical system of any one of examples 38-39, wherein the retention member is cylindrical.
41. The surgical system of any one of examples 38-40, wherein the driving member is monolithic.
42. The surgical system of any one of examples 38-41, wherein the drive element comprises one element selected from a set screw and a pin.
43. The surgical system of any one of examples 38-42, wherein the driving member includes a planar surface at least partially defining the opening, the planar surface facing the axis and being disposed at an angle between one and thirty degrees relative to the axis.
44. The surgical system of example 43, wherein the planar surface is disposed at an angle between five and fifteen degrees relative to the axis.
45. The surgical system of any one of examples 43 and 44, wherein the driving member further comprises a bottom surface at least partially defining the opening, the bottom surface being disposed opposite and facing the planar surface, and wherein a distance between the bottom surface and the axis of the driving member decreases as the driving member extends from a proximal end to a distal end.
46. The surgical system of example 45, wherein the retention member in the second position is spaced from the bottom surface of the opening and in contact with the planar surface and received in the groove of the surgical accessory tool.
47. The surgical system of any one of examples 38-46, wherein the surgical assembly further comprises a release collar slidably coupled to the driving member and a release biasing member disposed between the release collar and the driving member to bias the release collar away from the retention member, and wherein the release collar is slidable to abut the retention member to move the retention member to the second position in response to a force applied to the release collar sufficient to overcome opposing forces of the release biasing member and the retention biasing member.
48. The surgical system of example 47, wherein the release biasing member is disposed distal the retention member.
49. The surgical system of any one of examples 38-48, wherein the opening is further defined as a first opening and the driving member further defines a second opening symmetrically oriented relative to the first opening such that the retention member is disposed in the first and second openings.
50. The surgical system of any one of examples 38-49, wherein the driving member further defines a cavity in communication with the opening, and wherein the retention biasing member is disposed at least partially in the cavity of the driving member.
51. The surgical system of example 50, wherein the cavity of the driving member extends parallel to the axis of the driving member.
52. The surgical system of any one of examples 50-51, wherein the lock assembly further comprises an engagement pin disposed at least partially within the cavity and at least partially within the opening between the retention biasing member and the retention member, with the engagement pin adapted to transfer force from the retention biasing member to the retention member.
53. The surgical system of any one of examples 50-52, wherein the cavity is further defined as a first cavity and wherein the retention biasing member is further defined as a first retention biasing member, and wherein the driving member further defines a second cavity symmetrically oriented relative to the first cavity about a reference plane that extends through the axis, and wherein the lock assembly comprises a second retention biasing member disposed in the second cavity of the driving member, with the second biasing member also configured to urge the retention member toward the first position.
54. A surgical system comprising:
   a surgical accessory tool including at least one flat region; and
   a surgical assembly adapted for removable coupling with the surgical accessory tool,
   the surgical assembly comprising,
   a driving member extending along an axis from a proximal end to a distal end, the driving member being rotatable about an axis and configured to transfer torque to the surgical accessory tool, the driving member defining:
      (i) a receiving bore extending along the axis for receiving the surgical accessory tool, and
      (ii) an opening extending transverse to and partially into the receiving bore,
   a lock assembly coupled to the driving member to axially secure the surgical accessory tool to the driving member, the lock assembly comprising,
   a retention member partially disposed in the opening of the driving member for movement between a plurality of retention member positions including,
   a first position where a first volume of the retention member is disposed in the receiving bore of the driving member, and
   a second position where a second volume of the retention member is disposed in the receiving bore of the driving member, with the second volume being less than the first volume, and with a portion of the retention member abutting the flat region of the surgical accessory tool to axially secure the surgical accessory tool to the driving member, and
   a retention biasing member coupled to the driving member and configured to urge the retention member towards the first position.
55. The surgical system of example 54, wherein the surgical assembly further comprises a housing, and wherein at least a portion of the driving member is disposed within the housing.
56. The surgical system of example 55, further comprising a surgical driver having a first coupler, and wherein the housing comprises a second coupler adapted for removable attachment to the first coupler of the surgical driver.
57. The surgical system of example 54, further comprising a surgical driver having a first coupler, and wherein the driving member comprises a second coupler adjacent the proximal end of the driving member adapted for removable attachment to the first coupler of the surgical driver.
58. The surgical system of any one of examples 55-57, wherein the retention member is cylindrical.
59. The surgical system of any one of examples 55-58, wherein the driving member is monolithic.
60. The surgical system of any one of examples 55-59, wherein the retention member in the first position is disposed axially distal to the retention member in the second position.
61. The surgical system of any one of examples 55-60, wherein the driving member includes a planar surface at least partially defining the opening, the planar surface facing the axis and being disposed at an angle between one and thirty degrees relative to the axis.
62. The surgical system of example 61, wherein the planar surface is disposed at an angle between five and fifteen degrees relative to the axis.
63. The surgical system of any one of examples 55-62, wherein the driving member further comprises a bottom surface at least partially defining the opening, the bottom surface being disposed opposite and facing the planar surface, and wherein a distance between the bottom surface and the axis of the driving member decreases as the driving member extends from a proximal end to a distal end.
64. The surgical system of example 63, wherein the retention member in the second position is spaced from the bottom surface of the opening and in abutting contact with the planar surface and the flat region of the surgical accessory tool.
65. The surgical system of any one of examples 55-63, wherein the lock assembly further comprises an engagement collar disposed at least partially around the driving member between the retention biasing member and the retention member, with the engagement collar adapted to transfer force from the retention biasing member to the retention member.
66. The surgical system of any one of examples 55-65, further comprising a release collar slidably coupled to the driving member and a release biasing member disposed between the release collar and the driving member to bias the release collar away from the retention member, and wherein the release collar is slidable to abut the retention member to move the retention member to the second position in response to a force applied to the release collar sufficient to overcome opposing forces of the release biasing member and the retention biasing member.
67. The surgical system of example 66, wherein the release biasing member is disposed distal the retention member.
68. The surgical system of example 66, wherein the release biasing member is disposed proximal the retention member.
69. The surgical system of any one of examples 55-68, wherein the opening is further defined as a first opening and the driving member further defines a second opening symmetrically oriented relative to the first opening such that the retention member is disposed in the first and second openings.
70. The surgical system of any one of examples 55-69, wherein the driving member further defines a cavity in communication with the opening, and wherein the retention biasing member is disposed at least partially in the cavity of the driving member.
71. The surgical system of example 70, wherein the cavity of the driving member extends parallel to the axis of the driving member.
72. The surgical system of any one of examples 51-71, wherein the lock assembly further comprises an engagement pin disposed at least partially within the cavity and at least partially within the opening between the retention biasing member and the retention member, with the engagement pin adapted to transfer force from the retention biasing member to the retention member.
73. The surgical system of any one of examples 51-72, wherein the cavity is further defined as a first cavity and wherein the retention biasing member is further defined as a first retention biasing member, and wherein the driving member further defines a second cavity symmetrically oriented relative to the first cavity about a reference plane that extends through the axis, and wherein the lock assembly comprises a second retention biasing member disposed in the second cavity of the driving member, with the second biasing member also configured to urge the retention member toward the first position.

## Claims

1. A surgical assembly for coupling to a surgical accessory tool, the surgical assembly comprising:
a driving member extending along an axis from a proximal end to a distal end, the driving member being rotatable about an axis and configured to transfer torque to the surgical accessory tool, the driving member defining:
(i) a receiving bore extending along the axis for receiving the surgical accessory tool,
(ii) an opening extending transverse to and partially into the receiving bore, and
(iii) a cavity in communication with the opening; and
a lock assembly for securing the surgical accessory tool to the driving member, the lock assembly comprising,
a retention member partially disposed in the opening of the driving member for movement between a plurality of retention member positions including,
a first position where a first volume of the retention member is disposed in the receiving bore of the driving member, and
a second position where a second volume of the retention member is disposed in the receiving bore of the driving member, with the second volume being less than the first volume, and
a retention biasing member at least partially disposed in the cavity of the driving member and configured to urge the retention member towards the first position for engaging the surgical accessory tool.

2. The surgical assembly of claim 1, further comprising a housing, wherein at least a portion of the driving member is disposed within the housing, wherein the housing comprises a coupler configured to be removably attached to a surgical driver.

3. The surgical assembly of any one of claims 1-2, further comprising a drive element coupled to the driving member and disposed at least partially within the bore of the driving member opposite the axis from the opening of the driving member and configured to engage a flat region of the surgical accessory tool to transmit torque to the surgical accessory tool.

4. The surgical assembly of claim 3, wherein the drive element comprises one element selected from a set screw and a pin.

5. The surgical assembly of any one of claims 1-4, wherein the retention member in the first position is disposed axially distal to the retention member in the second position.

6. The surgical assembly of any one of claims 1-5, wherein the driving member includes a planar surface at least partially defining the opening, the planar surface facing the axis and being disposed at an angle between one and thirty degrees relative to the axis.

7. The surgical assembly of claim 6, wherein the planar surface is disposed at an angle between five and fifteen degrees relative to the axis.

8. The surgical assembly of any one of claims 6 and 7, wherein the driving member further comprises a bottom surface at least partially defining the opening, the bottom surface being disposed opposite and facing the planar surface, and wherein a distance between the bottom surface and the axis of the driving member decreases as the driving member extends from a proximal end to a distal end.

9. The surgical assembly of claim 8, wherein the retention member in the second position is spaced from the bottom surface of the opening and in contact with the planar surface.

10. The surgical assembly of any one of claims 1-9, wherein the cavity of the driving member extends parallel to the axis of the driving member.

11. The surgical assembly of any one of claims 1-10, wherein the lock assembly further comprises an engagement pin disposed at least partially within the cavity and at least partially within the opening between the retention biasing member and the retention member, with the engagement pin adapted to transfer force from the retention biasing member to the retention member.

12. The surgical assembly of any one of claims 1-11, further comprising a release collar slidably coupled to the driving member and a release biasing member disposed between the release collar and the driving member to bias the release collar away from the retention member, and wherein the release collar is slidable to abut the retention member to move the retention member to the second position in response to a force applied to the release collar sufficient to overcome opposing forces of the release biasing member and the retention biasing member.

13. The surgical assembly of claim 12, wherein the release biasing member is disposed distal the retention member.

14. The surgical assembly of any one of claims 1-13, wherein the opening is further defined as a first opening and the driving member further defines a second opening symmetrically oriented relative to the first opening such that the retention member is disposed in the first and second openings.

15. The surgical assembly of any one of claims 1-14, wherein the cavity is further defined as a first cavity and wherein the retention biasing member is further defined as a first retention biasing member, and wherein the driving member further defines a second cavity symmetrically oriented relative to the first cavity about a reference plane that extends through the axis, and wherein the lock assembly comprises a second retention biasing member disposed in the second cavity of the driving member, with the second biasing member also configured to urge the retention member toward the first position.
